# EUROPEAN PATENT SPECIFICATION

(11) **EP 2 349 189 B1**
(45) Date of publication and mention of the grant of the patent: **05.08.2015**
(21) Application number: 09756129.4
(22) Date of filing: 13.11.2009
(51) Int. Cl.: A61K 8/25, A61K 8/36, A61Q 11/00, A61K 8/81, A61K 8/19, A61K 8/22, A61K 8/24

(54) **BIOMIMETIC ORAL CARE COMPOSITION**
BIOMIMETISCHES MUNDPFLEGEZUSAMMENSETZUNG
COMPOSITION DE SOIN ORAL BIOMIMÉTIQUE

(30) Priority: 14.11.2008 EP 08019931
(43) Date of publication of application: 03.08.2011
(73) Proprietor: Swissdent Cosmetics AG, 8001 Zürich (CH)
(72) Inventor: VELKOBORSKY, Vaclav, CH-8008 Zürich (CH); GHOSH, Sankha, CH-8001 Zürich (CH)
(74) Representative: Grimm, Siegfried
(86) International application number: PCT/CH2009/000360
(87) International publication number: WO 2010/054494

(56) References cited:
- WO-A1-2008/068149
- US-A- 5 833 957
- US-A- 5 858 333
- US-A1- 2006 110 340
- US-B1- 6 482 395

## Description

The invention relates to oral care compositions, in particular a toothpaste, methods of manufacturing such compositions and methods of using such compositions.

The routine brushing of teeth after each meal is a customary practice; its purpose is twofold. First, brushing is considered an excellent form of preventive medicine. It is well established that continuous care by brushing will significantly extend the useful dental life for the practitioner, as both the incidence of dental demineralization and gum disease are reduced. For this purpose, the term "oral hygiene" is chosen. Second, and more often the primary driving force, is the desire to obtain a bright smile, full of shining white teeth. For this purpose, the term "teeth whitening" is chosen.

The term "biomineralization" is known in the field. It is a process in which living organism provides a chemical environment that controls the nucleation and growth of unique mineral phases. Often these materials exhibit hierarchical structural order, leading to superior physical properties, not found either in their inorganic counterparts or in synthetic materials. The abalone shell is one of many classic examples. Mollusk shells are composite materials, where small amounts of organic material guide the nucleation of calcium carbonite into precise crystal polytypes and growth habits. The resulting brick-and-mortar architecture of abalone nacre lends it a strength far exceeding that of inorganic calcium carbonate.

The tooth bud (also called the tooth germ) is an aggregation of cells that eventually forms a tooth involving several stages of biomineralization. The formation of a tooth comprises of its three parts which are enamel, dentin and cementum. Enamel formation is called amelogenesis and occurs in the crown stage of tooth development. "Reciprocal induction" governs the relationship between the formation of dentin and enamel; dentin formation must always occur before enamel formation. Generally, enamel formation occurs in two stages: the secretory and maturation stages. Proteins and an organic matrix form partially mineralized enamel in the secretory stage; the maturation stage completes enamel mineralization. In the secretory stage, ameloblasts release enamel proteins that contribute to the enamel matrix, which is then partially mineralized by the enzyme alkaline phosphatase. The appearance of this mineralized tissue, which occurs usually around the third or fourth month of pregnancy, marks the first appearance of enamel in the body. Ameloblasts deposit enamel at the location of what become cusps of teeth alongside dentin. Enamel formation then continues outward, away from the centre of the tooth. In the maturation stage, the ameloblasts transport some of the substances used in enamel formation out of the enamel. Thus, the function of ameloblasts changes from enamel production, as occurs in the secretory stage, to transportation of substances. Most of the materials transported by ameloblasts in this stage are proteins used to complete mineralization. The important proteins involved are amelogenins, ameloblastins, enamelins, and tuftelins. By the end of this stage, the enamel has completed its mineralization. Dentin formation, known as dentinogenesis, is the first identifiable feature in the crown stage of tooth development. The different stages of dentin formation result in different types of dentin: mantle dentin, primary dentin, secondary dentin, and tertiary dentin. Odontoblasts, the dentinforming cells, differentiate from cells of the dental papilla. They begin secreting an organic matrix around the area directly adjacent to the inner enamel epithelium, closest to the area of the future cusp of a tooth. The organic matrix contains collagen fibers with large diameters (0.1-0.2 µm in diameter). The odontoblasts begin to move toward the center of the tooth, forming an extension called the odontoblast process. Thus, dentin formation proceeds toward the inside of the tooth. The odontoblast process causes the secretion of hydroxyapatite crystals and mineralization of the matrix. This area of mineralization is known as mantle dentin and is a layer usually about 150 µm thick. Whereas mantle dentin forms from the preexisting ground substance of the dental papilla, primary dentin forms through a different process. Odontoblasts increase in size, eliminating the availability of any extracellular resources to contribute to an organic matrix for mineralization. Additionally, the larger odontoblasts cause collagen to be secreted in smaller amounts, which results in more tightly arranged, heterogeneous nucleation that is used for mineralization. Other materials (such as lipids, phosphoproteins, and phospholipids) are also secreted. Secondary dentin is formed after root formation is finished and occurs at a much slower rate. It is not formed at a uniform rate along the tooth, but instead forms faster along sections closer to the crown of a tooth. This development continues throughout life and accounts for the smaller areas of pulp found in older individuals. Tertiary dentin, also known as reparative dentin, forms in reaction to stimuli, such as attrition or dental caries. Cementum formation is called cementogenesis and occurs late in the development of teeth. Cementoblasts are the cells responsible for cementogenesis. Two types of cementum form: cellular and acellular. Acellular cementum forms first. The cementoblasts differentiate from follicular cells, which can only reach the surface of the tooth's root once Hertwig's Epithelial Root Sheath (HERS) has begun to deteriorate. The cementoblasts secrete fine collagen fibrils along the root surface at right angles before migrating away from the tooth. As the cementoblasts move, more collagen is deposited to lengthen and thicken the bundles of fibers. Noncollagenous proteins, such as bone sialoprotein and osteocalcin, are also secreted. Acellular cementum contains a secreted matrix of proteins and fibers. As mineralization takes place, the cementoblasts move away from the cementum, and the fibers left along the surface eventually join the forming periodontal ligaments. Cellular cementum develops after most of the tooth formation is complete and after the tooth occludes (in contact) with a tooth in the opposite arch. This type of cementum forms around the fiber bundles of the periodontal ligaments. The cementoblasts forming cellular cementum become trapped in the cementum they produce.

It is still not possible to obtain such functional, hierarchical arrangements at these length scales in apatite materials of human design.

The aim of "biomimetics" is to mimic the natural way of producing minerals such as apatites. Many man-made crystals require elevated temperatures, pressures and strong chemical solutions whereas the organisms have long been able to lay down elaborate mineral structures at ambient temperatures. Often the mineral phases are not pure but are made as composites which entail an organic part, often protein, which takes part in and controls the biomineralization. These composites are often not only as hard as the pure mineral but also tougher, as at last, the micro-environment controls biomineralization.

The enamel layer of a tooth is composed of HCA mineral crystals that create a somewhat porous surface. Therefore, it is susceptible towards acid dissolution at the surface, which is called "surface demineralization", as well as it allows the acid to permeate to the deeper structure/subsurface to cause "subsurface demineralization". The main composition of dentin and cementum is ceramic HCA, which is rather softer and amorphous in nature. Demineralization of dentin and cementum is classified here as a part of subsurface demineralization.

The oral plaque as well as dental plaque, which stem from the food and drink materials that a person consumes on daily basis, reside gum, gingival, buccal surfaces of teeth, micro cavities on the teeth, and tongue. These plaques are mainly composed of polysaccharides and proteins which act as potential host for bacterial colonies and microbial organisms. Two most important bacteria, found in plaque, are lactobacilli and streptococcus mutans that undergo an anaerobic acidic respiration which yields organic acids, such as malic acid, that stipulates significant acid dissolution of HCA from the enamel and dentin, and volatile sulfur containing compounds (VSC) that causes bad breath. Acid dissolution of HCA is the principle cause for dental caries, periodontal disease and tooth hypersensitivity problem, and also stipulates tooth discoloration as well as gingival inflammation, and thus an unavoidable threat for overall tooth health. Demineralization of cementum, which may occur due to deep inflammation at the root, leads to severe orthodontic diseases/problems, and subsequently loss of the tooth. It is also known that the saliva constantly attempts to remineralize the demineralized parts of the teeth; but this is considered an insufficient process.

Dental calculus is a calcified or calcifying mass which is generated from dental plaque. In its more advanced state, it consists of an organic matrix, representing about 20% of the deposit and containing carbohydrates, proteins, bacteria, cellular debris, and other organic materials. The major portion of calculus is inorganic. This portion contains calcium, phosphorus, magnesium and lesser amounts of other mineral elements. The calcium and phosphorus are present primarily in the form of various calcium phosphate species, the most common of which is hydroxyapatite (HCA). There are at least two well-recognized stages in calculus formation. First, the organic matrix or mucilaginous plaque (individually or simultaneously) deposits upon the tooth enamel or cementum, and may spread to adjacent areas in the oral cavity. At this stage, vigorous oral hygiene, especially tooth brushing, can remove much of the deposit and effectively reduce the amount of calculus subsequently found. In the second stage, alterations occur within the plaque which initiate mineralization. The exact mechanism whereby mineralization occurs has not yet been determined. Plaque tends to accumulate, selectively, calcium and phosphate. In one theory, it has been suggested that it is the activity of the microorganisms within the plaque which leads directly to the formation of dental calculus. Of the various types of bacteria present, the Leptothrix and Actinomyces appear to have been implicated most directly as being involved in both intercellular and extracellular mineralization. In another theory, it has been proposed that under appropriate conditions the mucilaginous matrix of the dental plaque is so altered as to promote mineralization. Here, too, the role of bacteria appears to be important; it has been observed that mineralization can start near the bacteria and that the bacteria are able to manufacture materials which are able to initiate the formation of insoluble calcium salts.

The enamel layer of a tooth is naturally an opaque white or slightly off-white color. It is this enamel layer that can become largely stained or discolored. It is believed that this porous nature of the enamel layer is what allows staining agents and discoloring substances to permeate the enamel and discolor the tooth. Surface demineralization further assists in permeating the staining substances.

Further, there is an increasing use of electric tooth brushes, in particular of electric toothbrushes using sonic vibration.. Such sonic care technology offers an excellent tooth brushing engineering, over the manual brushing methodology, in more efficient cleaning and polishing the teeth, removing the dental plaque, and reducing the S. mutans counts. However, toothpastes available today cause a potential frictional damage to the enamel of the tooth when applied with an electric, in particular sonic care tooth brush. Such tooth then becomes highly susceptible to suffer from hypersensitivity and catch stains.

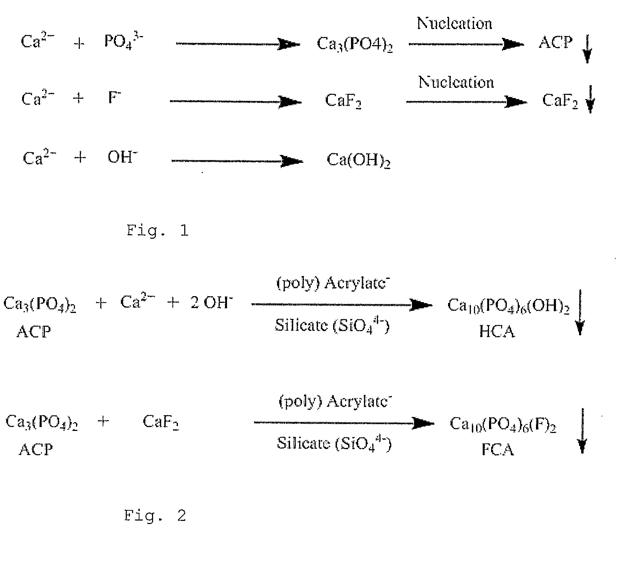

Biomimetics underpins an emerging novel approach in modern cosmetic dentistry towards replenishing HCA on dentin, enamel and cementum at subsurface level, since it offers a convincing remedy to the acid dissolution problem, remineralizes the demineralized parts of tooth, occludes the dentin tubules and micro-holes in enamel, prevents formation of dental carious lesions, eliminates dental hypersensitivity and periodontal diseases, subsides dental discoloration, and enhances natural whiteness as well as brightness of the teeth. In the context of this invention, the term "Dental Biomimetics" is used. Dental biomimetics particularly relates to the mineralization / re-mineralization of teeth. The chemistry underlying dental biomimetics is believed to be a two step process. In step one, hydrated calcium reacts with phosphate, fluoride and hydroxyl ions in aqueous medium of saliva, to produce calcium phosphate, calcium fluoride and calcium hydroxide, respectively. Calcium phosphate is insoluble in water provided that the pH of the medium is higher than 6, under which condition the produced calcium phosphate molecules agglomerate at the dental surface and subsurface to stipulate nucleation of nano-grains/crystallites that subsequently deposit amorphous calcium phosphate ACP thereon. Likewise, deposition of insoluble calcium fluoride takes place thereon. The chemical process in "step one" is further demonstrated in Fig 1. In step two, calcium hydroxide, whose sustenance requires alkaline pH higher than 7.05 at least of the medium, and calcium fluoride, react with the ACP, catalyzed by silicate ions and acrylate polymer ions, to produce hydroxyl calcium apatite (HCA) and fluoro calcium apatite (FCA), respectively, with physiochemical properties including structure, crystal/molecular packing and physical hardness that closely mimic to those of tooth. Silicate and acrylate polymer ions are believed to initiate the reaction transforming ACP to apatite materials and the guidance in crystal packing / molecular association during the formation of apatite materials with complex structure, in step two, such that the newly formed apatite materials mimic the structure and physical hardness of the tooth, and hence biomimetic mineralization of tooth. The chemical process interplaying in "step 2" is further explained in Fig 2.

US6770265 discloses a two-step application of dual-component solutions of aqueous calcium salts and phosphate salts, having pH of 2.5-5, to bonds between tooth-surface (i.e. dentin) and glass-ionomer cements with more enhanced adhering force and stability for the treatment of filling dental cavity. The low pH in this process is necessary to remove the smear layer as to establish bonds with deeper tooth-structure. This is similar to acid-itching process in the treatment of dental cavity. This invention teaches that calcium and phosphate ions greatly facilitate the ion-exchange reactions between glass-ionomers and tooth-structure, and that calcium and phosphate ions are susceptible to stipulate ion-exchange reaction with HCA of dentin and enamel. However, such a low pH disfavors formation and precipitation of ACP, which is necessary to form HCA and more importantly, a regular use of this composition will cause a severe acid dissolution of HCA. Therefore, compositions disclosed in this document are considered unsuitable for oral care, teeth whitening and in particular, dental mineralization/dental biomimetics.

US4177258, US4183915 and US4348381 provide a re-mineralizing solution containing supersaturated concentrations of calcium ions, phosphate ions and a fluoride source stabilized by the presence of an anti-nucleating agent such as diamine tetramethylenephosphonic acid, ethylenediamine tetramethylenephosphonic acid and 2-phosphonobutane-tricarboxylic acid-1,2,4, or the water-soluble salts thereof. Further, the disclosed solutions are preferably adjusted to the neutral pH range where it is alleged to most effectively remineralize sub-surface lesions. Even though the anti-nucleating agent would be expected to stabilize the solution, equilibrium of the supersaturated concentrations is still found difficult to maintain and avoid precipitation of hydroxyapatite and changes in the pH of the solution. Furthermore, an anti-nucleating agent largely ceases the precipitation of ACP in oral environment, which is very undesirable towards bio-mineralization. Thus, compositions disclosed in this document are considered unsuitable for oral care, teeth whitening and in particular, dental mineralization/dental biomimetics.

US4083955 and US4397837 disclose compositions and methods for re-mineralizing by consecutive treatment of the tooth surface with separate solutions containing calcium ions and phosphate ions. Further, fluoride ions may be present in the phosphate solution. By sequentially and separately applying calcium and phosphate ions, it is speculated that high concentrations of the ions penetrate into the tubules whereby they precipitate as calcium phosphate salts. Since dental caries begins as a subsurface demineralization of the dental enamel, subsurface remineralization arrests and repairs the carious lesion before any permanent structural damage to the tooth occurs. The invention thus addresses the important issue of subsurface re-mineralization rather than surface re-mineralization, and attempts to overcome the disadvantages of US No. 3 679 360 (which aimed to deposit ACP on roughened enamel surface). This invention, at least, suffers from the inconvenience of a plurality of sequential applications. Thus, compositions disclosed in this document are found unsuitable for oral care, teeth whitening and in particular, dental mineralization/dental biomimetics.

US4606912 discloses mouthwash solutions capable of re-mineralizing lesions in teeth by forming an aqueous solution containing a source of calcium ions and a chelating agent for calcium ions, causing the chelation of at least 50% of the calcium ions and subsequently adding a source of phosphate ions to the aqueous solution, as well as the manufacturing thereof. Again, it is believed that the addition and necessary control of the amount of chelating agent makes the concept impractical and thus unsuitable for oral care, teeth whitening, and in particular, dental mineralization/dental biomimetics.

US5858333 and US5833957 both disclose a two-part oral care composition in which calcium with another divalent metal in 7.52-4:1 weight-ratio and phosphate ions are kept separate, until dispensed and combined for treating the teeth. According to these documents, the presence of bivalent metal ions is alleged to allow sufficient time to Calcium and Phosphate ions for diffusing into the subsurface of enamel and dentin by supposedly preventing the rapid precipitations of Calcium Phosphate on the tooth-surface immediately after dispensing. However, the water solubility and hydrated ionic-radius of the phosphate and fluoride salts of these bivalent metals are very close to those of calcium phosphate and calcium fluoride, respectively. Therefore, on contrary to accomplish with controlled and delayed precipitation of Calcium Phosphate and Calcium fluoride onto subsurface of tooth, these bivalent metal ions will take part both in ion-diffusion as well as coprecipitations of their phosphate and fluoride salts. In other words, the presence of these bivalent metal ions has no role in delaying the precipitation of calcium phosphate and fluoride, but instead they will consume phosphate and fluoride ions to precipitate their phosphate and fluoride salts, along with these salts of calcium, on the surface on enamel. Therefore, the disclosed compositions in these two documents are considered not efficient for tooth mineralization at subsurface level. Further, it is well known in the field that carboxyl methyl cellulose (CMC, is incompatible with bi- and higher valent metal ions. Typically, a CMC based formulation containing bi- and higher valent metal ions shows rapid synerisis / phase separation yielding unstable product with very limited shelf-life. As a result, the examples from 11 to 16, 18 to 21, 25 to 28 and 31 disclosed in US5858333, and example 6 disclosed in US5833957 are unstable formulations. Furthermore, disclosed compositions in US5833957 claim to have a pH about 5.5 which is not sufficient to perform mineralization because transformation of ACP to HCA requires hydroxyl ions that can sustain in alkaline pH medium. Thus, compositions disclosed in these documents are found unsuitable for oral care, teeth whitening and in particular, dental mineralization / dental biomimetics.

US6482395 discloses an edible solid product comprising of discrete cationic agglomerate component of water-soluble or partially water-soluble calcium salts and at least one nontoxic divalent metal salt other than a calcium salt, and a discrete agglomerate component of water-soluble orthophosphate salts. The composition is claimed to simultaneously release these ions, upon contact to water, and hence to mineralize tooth at subsurface level As discussed above, the presence of these divalent metal ions has no role in delaying the precipitation of calcium phosphate and fluoride, but instead they consume phosphate and fluorides ions to precipitate their phosphate and fluoride salts, along with these salts of calcium, on the surface of enamel. Therefore, the compositions disclosed in this document do not provide a suitable means for efficient tooth mineralization at subsurface level. Thus, compositions disclosed in this document are considered unsuitable for oral care, teeth whitening and in particular, dental mineralization / dental biomimetics.

US20060110340 discloses a two part oral care composition comprising a calcium ion releaser composition, a phosphate ion releaser composition containing an orthophosphate and a peroxide. According to this document, upon application to the tooth, tooth whitening (due to bleaching) and tooth mineralization (due to deposition of calcium phosphate) occur. Although alkaline pH is expected to stipulate tooth mineralization, absence of an appropriate care / mechanism to control and delay the precipitation of ACP will lead to large amount of undesirable surface mineralization instead of subsurface mineralization, while the latter is of paramount importance in this context, as addressed by US4083955, US4397837, US4177258, US4183915, US4348381, US5858333 and US5833957, as well. Furthermore, the gel disclosed in example 2 of this document, suffers from instability in composition and poor shelf-life, particularly the Calcium containing gel composition, which uses CMC. Thus, compositions disclosed in this document are found unsuitable for dental mineralization / biomimetics.

WO2008/068149 discloses a two part oral care composition comprising a first and a second composition useful for tooth mineralization. Said first composition contains an insoluble calcium salt, other than calcium phosphate, with diameter 0.4-4 nm. Said second composition contains a water-soluble phosphate salt.

The prior art discussed above refers to "step one" (fig. 1), but does not consider "step two" (fig. 2). Step two and its requisite chemical reagents, are considered the most important step involved in tooth mineralization because the apatite materials are formed in this stage. Consequently, the prior art documents do not provide clear insight to the chemical processes and their reagents that underlie "tooth mineralization", which is the targeted topic of the present invention. Unless the chemical structure and physical hardness of the newly replenished apatite materials (guest) closely match to those of tooth (host), a rapid wear-out between guest and host materials is inevitable, and such mineralization is considered to be undesirable, inefficient and not biomimetic. Therefore, it is a prime object of the present invention to guide the formation and growth of apatite materials, onto subsurface and surface of tooth, such that they would considerably match / mimic to the physiochemical properties, including structure, molecular / crystal packing, and physical hardness (in particular scratch hardness) of the tooth as to efficiently and contiguously replenish new apatite materials to the tooth (tooth structure) and thus efficiently accomplish with tooth mineralization in a biomimetic fashion.

Further, prior art has not considered an important consequence / issue which is associated with dental re-mineralization: It increases the possibility of mineralizing the dental plaque, which promotes building up the dental calculus/tartar. Therefore, precaution must be taken care of, which is also an important object of the present invention.

Thus, there is a need for improved compositions for oral care, in particular of improved toothpastes. It is an object of the present invention to provide such compositions and to mitigate at least some of the drawbacks of the presently known compositions. In particular, it is an aim of the present invention to provide a composition that has a pronounced cosmetic and / or therapeutic effect on teeth or teeth structures, by engineering biomimetic subsurface mineralization. It is a further aim to provide an improved oral care composition adapted to the uses of electrical tooth-brushes (in particular sonic vibration toothbrushes).

These objectives are achieved by a composition as defined in claim 1, manufacturing methods as disclosed in claim 15 and methods of use as defined in claim 18. Further aspects of the invention are disclosed in the specification and independent claims, preferred embodiments are disclosed in the specification and the dependent claims.

The present invention will be described in more detail below. It is understood that the various embodiments, preferences and ranges may be combined at will. Further, depending of the specific embodiment, selected definitions, embodiments or ranges may not apply.

Unless otherwise stated, the following definitions shall apply in this specification:

The term containing shall, in the context of this invention, also include the meaning of comprising or consisting of.

The term treatment (e.g. of a condition or a disorder) shall, in the context of this invention, also include the prevention and / or delay of progression of said condition or disorder.

The term oral care shall, in the context of this invention, include dental biomimetics, oral hygiene and teeth whitening.

The term mineralizing ions shall, in context to the present invention, include such ions that are important for effectively mineralizing the teeth; this particularly includes calcium ion, phosphate ions, fluoride ion, hydroxyl ion, silicate ions and acrylate polymer releasing ions; both in hydrated and non-hydrated forms.

The term film forming agent shall, in the context of this invention, include agents capable of forming a solid thin film, covering the surface of tooth within a time period of less than 10 seconds. Advantageously, said film is permeable towards ion-diffusion of the mineralizing ions, and is removable by mechanical erosion (e.g. caused by brushing the teeth).

The terms "water soluble" "sparingly water soluble" and "water insoluble" have a well recognized meaning in the field; which also applies to this invention. In particular a compound of which at least 0.21 g (more specifically 0.25 g) thereof dissolves in 100 ml of water at 20°C is considered "water-soluble". In turn, compounds having a solubility of 0.21g to 0.0095 g per 100ml of water at 20°C are considered "sparingly water soluble" and compounds having solubility below 0.0095g in 100ml of water at 20°C are considered "water insoluble".

In a first aspect, the invention relates to an oral care composition comprising a first and a second composition wherein said first and second composition being maintained separate from each other until dispensed and combined for simultaneous application; said first composition contains a phosphate ion releaser having a water solubility of at least 2,1 g/l; said second composition contains a calcium ion releaser having a water solubility of at least 2,1 g/l said first and / or said second composition contains a film forming agent; said first and / or said second composition (preferably said first composition)contains an acrylate and / or poly-acrylate (collectively: "acrylate polymer"); said first and / or said second composition contains a silicate ion releaser; said first and / or said second composition optionally contains abrasive particles; and said first and / or said second composition (preferably said first composition) optionally contains a fluoride ion releaser. As it will become apparent from reading this specification, the inventive composition may additionally contain water and further additives as defined below.

It was surprisingly found that such oral care composition, formulated as a two-part composition, provides biomimetic mineralization to the subsurface of enamel and dentin, occludes the dental tubules and caries, cleans and whitens/brightens the teeth. Thus, such composition is reviving the overall dental health, efficiently removing oral plaque from the micro cavity and is lowering the tooth hypersensitivity. Such oral care composition is particular efficient when simultaneously applied through a sonic vibration toothbrush device. Further, the disclosed oral care composition is stable in storage.

The invention is explained in further detail below:
Without being bound to theory, it is believed that a simultaneous application of these two compositions (e.g. during brushing the teeth) results in multiple beneficial effects: a) It provides an in situ formation of nano-grains of amorphous calcium phosphate (ACP) on dental subsurface, dental tubules, caries and enamel surface. b) It is further believed that a mechanical vibration facilitates decreasing the porosity of ACP grains during subsurface nucleation, catalyzed by acrylate polymer ions and silicate ions present, resulting occlusion of the dentinal tubules, caries, micro-cavities and subsurface re-mineralization of tooth. c) It is also believed that the thin film administers controlled ion-diffusion through tooth-surface, eliminates undesired surface re-mineralization through hindering the excessive deposition of ACP on the surface and later removing the precipitation of ACP from the tooth surface, and increases the surface-area of contact that largely promotes the absorption-probability of the said mineralizing ions by the subsurface of tooth and hence largely accelerates the overall bio-mineralization at dental subsurface level. d) It is further believed that the film also accumulates the plaque, during brushing the teeth, on it, and hence eliminates the chance of undesirable mineralization of the plaque that gives rise to dental calculus. Brushing, in particular a sonic vibration brushing technique, easily removes the thin film.

As indicated above, the inventive oral care composition contains a first and a second composition which are maintained separate from each other until dispensed and combined for simultaneous application. It was found that re-mineralizing ions (in particular Calcium from, Phosphate and Fluoride ions) are advantageously kept from reacting during the storage of the composition. This may be accomplished by using a dual-compartment system wherein calcium ions are kept in a first composition (Ca-substrate) while the phosphate and fluoride (if present) ions are kept in a second composition (P-substrate), and they come in contact only when applied on teeth. Since delayed precipitation of ACP is more advantageous as it allows the ions to diffuse into the deeper structure (subsurface) of enamel and dentin, neither a prior mixing nor a sequential application of Ca and P substrate is necessary. This is considered a substantial advantage over the known oral care compositions.

Generally, the inventive composition contains such precursors of the calcium, phosphate, fluoride, acrylate polymer and silicate ions, which play key role in re-mineralization, and which readily release these ions as soon as in contact to water or an aqueous environment. Therefore highly water soluble compounds of these ions, (at least substantially water soluble compounds of these ions) are used as the precursor ingredients.

Concentration: It is generally known that ionic concentration plays a key role towards ion mobility and hence in ion-diffusion rate. Medium/moderate concentrations of the precursor/mineralizing ions are thus preferred. Suitable concentrations depend inter alia on the type of formulation and may be determined in routine experiments.

Phosphate ion releaser: Generally, any compound known in the field capable of releasing phosphate ions in a physiological environment (such as the salvia of the mouth), may be used. Suitable phosphate ion releasers are water soluble, in particular with a solubility of at least 2.1 g/l (20°C). Preferably, the phosphate ion releaser is selected from the group consisting of alkali metal- (e.g. potassium), urea-, and ammonium-phosphates. Generally, any phosphate ion releasing substance (i.e. a water soluble phosphate compound) known in the field may be applied. Suitable phosphate compounds are for example selected from the group consisting of alkali salts and ammonium salts of orthophosphoric acid, such as potassium, sodium or ammonium orthophosphate, mono-, di-, tri-potassium phosphate, mono-, di-, tri-sodium phosphate, urea phosphate, Tri-Methyl or Tri-Ethyl Ammonium Phosphate, Alkali Metal Salts of D-Glucose 6-phosphate, Alkali Metal Salts of Riboflavin 5'-monophosphate, with preference given to the above identified compounds. It is understood that either one or more phosphate ion releasing compound can be used. The amount of phosphate used in the P substrate may vary in a broad range and can be determined by simple routine experiments; suitable are, for example, 0.05 - 15 wt.-% of phosphate in said P-substrate.

Calcium ion releaser: Generally, any compound known in the field capable of releasing calcium ions in a physiological environment (such as the salvia of the mouth), may be used. Suitable calcium ion releasers are water soluble, in particular with a solubility of at least 2.1 g/l (20°C). Preferably, the calcium ion releaser is selected from the group consisting of calcium-glycerophosphate, -nitrate, -hydroxide, - hypochlorate, -bicarbonate, -peroxide, -silicate, - propionate. Generally, any Ca ion releasing substance (i.e. a water soluble Ca compound) known in the field may be applied. Suitable calcium compounds are for example selected from the group consisting of calcium nitrate, calcium oxide, calcium hydroxide, calcium bromide, calcium peroxide, calcium formate, calcium acetate, calcium malate, Calcium citrate malate, calcium chloride, calcium glycerophosphate, Calcium propionate, calcium acetate, calcium hypochlorite, Calcium lactate gluconate, calcium benzoate, calcium fumarate, calcium lactate, calcium butyrate, calcium isobutyrate, calcium maleate, calcium valerate. It is understood that either one or a mixture of Ca ion releasing compound can be used, with preference given to the above identified compounds. The amount of Ca used in the Ca subsystem may vary in a broad range and can be determined by simple routine experiments, suitable are, for example 0.05-15wt-% of Calcium in said Ca-subsystem.

Film forming agent: A broad range of compounds or compositions known in the field may be used as film forming agent, provided said compound / composition i) is capable of forming a film upon contact with a physiological environment (such as the salvia of the mouth); ii) the film thereby formed is thin, solid, quick-forming/setting, easily removable and ion-diffusion permeable. Consequently, gelling agents, which often form a liquid or semi-liquid film, are not considered "film forming agents" in the context of the invention. It is believed that the presence of one or more film forming agents significantly improves the ability of subsurface mineralization, as opposed to undesirable surface mineralization and is thus a key element of the present invention. The film forming agent(s) as defined herein positively influence shelf life and performance (in terms of oral hygiene and teeth whitening) of the present oral care formulation. The concept of forming temporarily a thin solid film is used to monitor and facilitate the ionic diffusion rate of the ions penetrating into the substructure of tooth. Formation of thin film largely increases the microscopic area of contact, also called true area of contact, which in one hand protects the tooth-surface from accumulating excessive deposition / precipitation of ACP, eliminating undesired surface mineralization, while on other hand it largely assists the covered tooth-surface to absorb the mineralizing ions due to expanded surface of absorption, and therefore a greater number such ions can then penetrate through a higher number of diffusion-channels into the deeper/inner tooth-structure. So, in this way, thin film largely enhances the diffusion probability of the mineralizing ions through the surface while lowering the precipitation of ACP on surface, and hence it largely favours the subsurface mineralization over the surface mineralization. A suitable solid thin film is easily removable by mechanical erosion via brushing, especially sonic vibration toothbrush. Film formation is very quick and requires only a few seconds (1-10 seconds) of drying time. The ACP's that have managed to get deposited on tooth surface are trapped under the film, while the further deposition of ACP takes place onto the surface of the film. Therefore, these ACP's are easily removed along with the film via brushing. To a further advantage, the thin film, due to its large surface-adhesion property, arrests and accumulates the plaque, which is then removed along with the film. Thus, it effectively reduces the possibility of mineralizing the plaque.

In a preferred embodiment, the invention relates to a composition as described herein, wherein said film forming agent is selected from the group of poly(meth)acrylate polymers. Poly (meth) acrylate polymers includes i) anionic polymers with methacrylic acid as a functional group ii) cationic polymers with methacrylate as a functional group, iii) neutral meth-acrylate copolymers. Meth-/acrylate copolymers with ammonium and alkali metal methacrylate functional groups are preferred. Further, one or a mixture of these polymers can be used. Particular suitable poly(meth)acrylates are known as EUDRAGIT® (such as Eudraglit RL 12,5, RL 30 D, RL 100, RL PO, RS 12,5, RS 30 D, RS 100, RS PO). For the avoidance of doubt, poly(meth)acrylate polymers are esters or salts of poly(meth)acrylic acid which must not be confused with acrylate polymer ions (which have important catalytic properties in "step 2"). The film forming agent is preferably incorporated in the P-substrate. The amount of film forming agent may vary over a broad range and can be determined by simple routine experiments; suitable are, for example, concentration ranges between 0.1 and 20 wt-%.

Acrylate polymer ion releasers: (also identified as "(Poly)acrylate ion releasers") Generally, any compound known in the field capable of releasing (poly)acrylates in a physiological environment (such as the salvia of the mouth), may be used. These ions are believed to catalyze and facilitate the formation of complex apatite materials of HCA and FCA with appropriate physiochemical properties, including structure, molecular/crystal packing and physical hardness that closely mimic to those of tooth, from ACP.

In a preferred embodiment, the invention relates to a composition as described herein, wherein said acrylate ion releaser and / or (poly)acrylate ion releaser (collectively: "acrylate polymer ion releaser") is selected from the group consisting of ammonium and metal acrylates and ammonium and metal polyacrylates.

In a further preferred embodiment, the invention relates to a composition as described herein, wherein said acrylate polymer ion releaser is Carbomer and / or Carbopol. They are preferably incorporated in the P substrate. It is believed that they show certain incompatibility with calcium, as well as with other bi- and higher valent cations, which greatly reduces the stability and shelf-life of the composition. The amount of acrylate polymer ion releaser agent used may vary in a broad range and can be determined by simple routine experiments, suitable are, for example, 0.05-5wt-% of acrylate polymer in the composition.

Silicate ion releasers: The invention relates to a composition as described herein, wherein said first and / or said second composition contains a silicate ion releaser. Silicate ion releases are believed to positively influence film formation of poly(meth)acrylates. Generally, any compound known in the field capable of releasing silicates in a physiological environment (such as the salvia of the mouth), may be used; this includes SiO4 (ortho-silicate) and other di- and polymer silicate ions. Suitable silicate compounds are for example selected from the group consisting of metal silicates such as alkali silicates and Calcium silicate. Calcium silicate is preferably added to the Ca substrate. It is understood that either one or a mixture of said silicate releasing compounds can be used. The amount of silicate used may vary in a broad range and can be determined by simple routine experiments; suitable are, for example, 0.05-5wt-% of silicate in the composition.

Fluoride ion releaser: The inventive oral care composition may contain a fluoride ion releaser and / or fluorophosphates, preferably present in said first composition. Generally, any inorganic or organic fluroide releasing substance (i.e. a water soluble fluoride compound) known in the field may be used. Suitable fluoride compounds are for example selected from the group consisting of alkali fluorides (such as sodium, potassium, lithium fluorides), tin fluoride, indium fluoride, zirconium fluoride, copper fluoride, nickel fluoride, palladium fluoride, fluorozirconates (such as sodium, potassium or ammonium fluorozirconate or tin fluorozirconate), fluorosilicates, fluoroborates, fluorostannites, organic fluorides (such as amine fluorides). Suitable fluophosphates are for example selected from the group consisting of alkali metal monofluorophosphates (such as sodium monofluorophosphate, lithium monofluorophosphate and potassium monofluorophosphate), ammonium monofluorophosphate, aluminum monofluorophosphate. Preferably, sodium monofluorophosphate is employed. It is understood that either one or a mixture of said fluoride or fluorophosphates compounds can be used. The amount of fluoride used may vary in a broad range and can be determined by simple routine experiments, suitable are, for example 0.01-5wt-% of fluoride in the composition. The fluoride ion releaser is preferably incorporated in said first composition (P-substrate). This avoids in a simple way formation of CaF2 during manufacturing and / or storage.

Abrasives: In a preferred embodiment, the invention relates to a composition as described herein, wherein said first and / or said second composition contains an abrasive (abrasive particles). Generally, any abrasive known in the field may be applied. Suitable abrasives are for example selected from the group consisting of silica (such as fumed silica, anhydrous silica, hydrated silica), Brushite, CaHPO₄·2H₂O, Kaolin, Aragonite, Calcite, white Pearl powder, Baking Soda, Calcium Carbonate, Acrylic glass (such as PM/MA) and Aluminium hydroxide. It is understood that either one or a mixture of said abrasive can be used. Abrasives are particular preferred when the oral composition is formulated as a tooth past or gel. Acrylic glass is preferred abrasive, in particular if the inventive composition contains oxygen and / or a chlorine releasing substances. This may be attributed to the oxidative stability of Acrylic glass. Brushite is a further preferred abrasive. This may be attributed to its ability to act as a precursor for HCA. In a preferred embodiment, the invention relates to a composition as described herein, wherein said abrasive particles have an average particle size of 50 - 200 µm, preferably 50 - 100 µm. The particle size of said dental abrasive (e.g. silica), is preferably in this range in order to accomplish with low radioactive dentin abrasion (RDA). This makes the composition particular suitable for sonic vibration-device application. It is also understood that various other particulate abrasives of the above identified particle size may replace any of the specifically identified abrasives in the oral care composition of the present invention. The invention thus also relates to an oral care composition containing one or more dental abrasives with an average particle size of 50 - 200 µm. The amount of abrasive used may vary in a broad range and can be determined by simple routine experiments, suitable are, for example 1-8 wt-%, preferably 1-7 wt-%, particularly preferably 1-5 wt-%, of abrasive in the composition. Suitable are further 3-7 wt-% of abrasive in the composition.

Additives: In a preferred embodiment, the invention relates to a composition as described herein, wherein said first and / or said second composition further contains one or more ingredients ("additives") selected from the group consisting of humectants, gelling agents, thickening agents, binding agents, detergents, colorants, perfumes, flavouring agents, pH regulators, nutritients, bleaching agents, preservatives, carriers. The additives, and suitable concentrations thereof, are known to the skilled person. Particular suitable additives are identified below.

Humectants: Humectants, may be either anhydrous (in case of substantially non-aqueous compositions) or hydrous (in case of aqueous compositions), and may be incorporated in said first and / or said second composition. If the composition is non-aqueous, then the humectants are preferably selected from the group consisting of anhydrous glycerol, anhydrous Propylene Glycol, Polyethylene Glycols (PEG 400, PEG 600, PEG 800), hydrogenated vegetable oils, hydrogenated polybutene, hydrogenated polyisobutene, hydrogenated polydecene, hydrogenated polyisodecene, and ethanol. Suitable concentrations of these humectants are in the range of 1-50% wt-%. If the composition is aqueous, then the humectants are preferably selected from the group consisting of water, glycerol, Propylene glycol, PEG, D-sorbitol. Suitable concentrations of these humectants are in the range of 1-60% wt-%.

Thickening agents: (also referred to as gelling agents, binders, viscosity enhancers): One or more binding agents (thickening or gelling agents) may be added to said first and / or said second composition. The selection of said binder inter alia depends on the water content of the compositions. If the composition is aqueous, a suitable binder is the one which is stable and compatible to give desired level of thickening property to the composition in presence of high electrolyte concentration (0.1-30%), at first place. Furthermore, it has be to stable and compatible with bivalent cations/metal ions, calcium ion in particular, as well, to produce a stable Ca-substrate with desired level of thickening property and reasonable shelf-life, pertinent to formulate end-use products.

Preferred binder can be chosen from an inorganic class and / or organic class of binders, or a suitable mixture thereof. Preferred concentration may vary over a broad range, 0.001 to 50%. In this context, a binder is indentified as a viscosity enhancing agent, which refers to any agent that, when applied in various concentrations in an aqueous or non-aqueous medium, results in the formation of stable gels that exhibit thixotropic properties.

The inorganic class comprises thickening silicas (hydrated, precipitated, fumed, etc.), colloidal silicas, alkali-earth metal aluminium silicates, natural and synthetic clays. Zeodent 165, Sident 22S, Syloblanc 34 and the like are proven useful thickening silica, with use level 0.1 to 40%. Non-limiting examples of the at least one viscosity-enhancing agent include magnesium aluminum silicates, smectite clays, and an amorphous clay mineral, such as allophone; two-layer type crystalline clay minerals, such as equidimensional crystal, kaolinite, and nacarite; elongate crystals, such as halloysites; three-layer type crystalline clay minerals, such as sodium montmorillonite, calcium montmorillonite, sauconite, vermiculite, nontronite, saponite, hectorite, and bentonite (Optigel); chain structure crystalline clay minerals, such as attapulgite, sepiolite, and palygorskite; sodium lithium magnesium silicate (Laponite) and mixtures thereof. For aqueous formulations, Laponite and Optigel, supplied by Rockwood additives, have proven promising, although they are not preferably used in the compositions wherein concentration of Ca is higher than 6%. For non-aqueous, Thixogel type of clay-gelling agents, supplied by Rockwood has proven very promising for formulating P- as well as Ca- substrate compositions. Thixogel based compositions have shown to work with high electrolyte (30%) as well as high concentration of calcium (15%). The use level of said clay gelling agents may vary between 1 and 15% depending on the compositions. For high electrolyte, as well as high calcium content compositions, which are mainly targeted for professional care products, the leading viscosity enhancer agent is one or a mixture of thickening silicas, either with a combination of clay gelling agents and / or organic class of thickener.

The organic class falls into two major families; non-ionic and ionic. Non-ionic class comprises (C₂₋₆ )alkylene oxide modified (C ₁₋₆ )alkylcellulose ethers, for instance, hydroxyethyl cellulose HEC (Natrosol and modified Natrosol type of gelling agents supplied by CP Kelco) hydroxypropyl cellulose, hydroxypropyl methylcellulose; Xanthan gum (Keldent from CP Kelco); Guar gum (calcium ions and / or electrolytes help gelling/thickening); gum Karaya; gum Tragacanth; Irish moss; gum Arabic; cornstearch; polyvinylpyrrolidone; Copolymers like Methyl vinyl ether/maleic anhydride copolymer (PVM/MA); polyethylene glycol (PEG) block polymers; etc. The use level may vary between 0.1 and 15% depending on the compositions.

The ionic class comprises anionic and cationic types of organic thickening agents. Anionic type includes new carbopols, especially the ones that are designed to be stable and compatible with electrolytes, for example Carbopol Ultrez 21 and Carbopol Aqua SF-1; Walen Gum (compatible with calcium); gellan gum; alkali metal carrageenan; etc. Cationic type includes guar hydroxypropyl trimonium chloride, and the like. The use level of the ionic binder may vary over a wide range between 0.1 and 10%.

It has been found that a combination of said inorganic and organic thickening agents, in 300:1 to 2:1 %-weight ratio, is advantageous in formulating practical compositions, for both aqueous based personal care (dental cream) and professional care (higher amount of calcium and phosphate contents), with reasonable stability, shelf life, desired functionalities, viscosity, density, rheological properties, surface texture, feeling, etc., which are important characteristics of an end-user product, ready to sell. However, it does not limit the scope to use either one of them alone within present invention. Further, selection of one or a group of said inorganic and organic binder is sensitive toward aqueous based (a) personal care (dental creams) compositions, that contain usually lower amount of electrolyte contents in P- and Ca- substrates Vs professional care compositions that contain usually higher amount of electrolyte contents in both P- and Ca- substrates, (b) P- Vs Ca- substrate and (c) if an acidic bleaching or an alkaline bleaching agent is to be incorporated either in both or in P- or in Ca- substrates. For non-aqueous and low aqueous based formulations, the selection of one or a group of binders is relatively less dicey. For aqueous and low water based personal dental care compositions disclosed herein, wherein the concentrations of calcium and phosphate ion releaser agents are below 6% and total electrolyte concentrations are below 12% in each substrates, thickening silica; Laponite, Optigel and Thixogel in clay gelling group; and colloidal alkali metal aluminum silicate from said inorganic binder/gelling agents; and non-ionic and anionic gelling (non-ionic are more preferred over the ionic ones) agents from said organic binder/gelling agents are preferred to be incorporated in P-substrate, while for Ca-substrate thickening silicas; if used, not more than 4% of Laponite and Optigel; and Thixogel from inorganic binder, and Hydroxyl ethyl cellulose (Natrosol 250 HH, Natrosol 250 H4, Natrosol 250 H, modified Natrosol, etc., supplied by CP Kelco), Xanthan gum (Keldent, supplied by CP Kelco), Guar gum from non-ionic organic thickener group; and walen gum from organic anionic thickener group are preferred. For aqueous compositions with even lower concentration of total electrolytes (less than 6%), said Carbopols and alkali metal carrageenan may be incorporated into P-substrate; but preferably not in Ca-substrate since they will affect the stability and shelf-life of Ca-substrate. For aqueous and low water based professional dental care compositions disclosed herein, wherein the concentrations of calcium and phosphate ion releaser agents are higher than 6% and total electrolyte concentrations are higher than 12% in each substrate, thickening silicas and Thixogel from inorganic binder class are preferred for both P- and Ca-substrate and Guar gum, Xanthan gum, low substituted Hydroxyl ethyl cellulose (M type Natrosol) and PEG block polymer are preferred from non-ionic class of binder are preferred for P-substrate, while for Ca-substrate Guar gum from non-ionic and welan gum from anionic organic binder are preferred.

Sodium or alkali metal salts of Carboxymethyl cellose CMC, Carbopol 2020 and Carbomers, which are widely used as thickening agents in Oral care products, are unsuitable to formulate the compositions within the scope of present invention. It is believed that this incompatibilty is due to the incompatibility with electrolytes and bi- and higher valent metal ions, in particular calcium ion (which is required to be incorporated in the Ca-substrate in order for desired level of tooth mineralization and whitening). Ca-substrate with concentration higher than 1 wt-% of calcium ion, and P-substrate with concentration of total electrolyte higher than 6 wt-%, which are formulated with CMC, Carbopol 2020 and Carbomers, have shown "syneresis" within between 2 weeks and 2 months after production, which is not pertinent to qualify for end-user product. Therefore, the present invention relates to oral care compositions as defined herein, wherein Carboxymethylcellulose salts are not present.

If the composition is non-aqueous or substantially non-aqueous, suitable gelling agents may be selected from the group consisting of polysorbates (such as Polysorbate 20, Polysorbate 60 and Polysorbate 80), a mixture of Ethyl Cellulose, Calcium Acetate, a mixture of N-vinylacetamide and sodium acrylate, a fatty acid mono-or poly- ester of a mono- or poly-glycol, mono- or polyglycerol and their amide or oxide derivative, such as Glyceryl MonoStearate, Ceto Stearyl Stearate, Ethylene Glycol MonoStearate, Poly Glyceryl Stearate, Glyceryl Mono Laurate, Glyceryl Mono Palmitate, Glyceryl Mono Oleate, Coco Mono Ethanolamide, Ethylene Glycol Di Stearate, Propylene Glycol Mono Stearate, PEG Mono Laurate, PEG Mono Stearate, Cetyl Palmitate, Stearyl Stearate, Coco Di Ethanolamide, Butyl Stearate, 2 Ethyl Hexyl Stearate, 2 Ethyl Hexyl Palmitate, , Sorbitan Mono Laurate, Sorbitan Mono Palmitate, Sorbitan Mono Stearate, Sorbitan Mono Oleate, Lanolin, Aloe Vera, etc.. Suitable concentrations for such gelling agents may vary in a broad range, typically 0.5-35 wt-% (excluding solvents). Such gelling agents may be combined with one or more suitable solvents, particularly selected from the group consisting of Propylene Glycol dicaprylate, Propylene Glycol dicaprate, Isopropyl Myristate and Isopropyl Palmitate.

In a preferred embodiment, the invention relates to a composition as described herein, wherein said thickening agent is selected from a mixture of first and second groups of compounds, where first group comprises solid esters such as Glyceryl Tribehenate / isostearate / eicosadioate, Glyceryl behenate / eicosadioate, Polyglyceryl-10 Behenate / eicosadioate, Polyglyceryl-10 stearate, etc., and the second group comprises liquid esters, hydrocarbons, glycerides, mineral oils, alcohols, glycols, polyglycols, glycerols, and silicones. This binder class is suitable for both non-aqueous and aqueous formulations. In aqueous and low water content formulations they act further as stabilizer of O/W and W/O.

In a preferred embodiment, the invention relates to a composition as described herein, wherein said thickening agent is selected from a group of wax materials comprising polawax, emulsifying wax, etc. They are suitable to both non-aqueous and aqueous formulations.

Detergents: A detergent (also referred to as surfactant), in particular a mild detergent, may be incorporated into said first and / or said second composition. It is also known in the field that a carrier, as defined herein, may function as a detergent. Suitable detergents are known in the field and include non-ionic, zwitterionic, cationic and anionic surfactants, with preference given to non-ionic and zwitterionic surfactants. It is understood that mixtures of detergents, as known in the field, may also be applied.

Preferred non-ionic surfactants are selected from the group consisting of Alkyl poly(ethylene oxide), Polysorbate esters (polysorbate 20, 60, 80), alkyl amine oxides, ether carboxylic acids, , Poloxamer, Cetyl Alcohol, Oleyl Alcohol, Cocamide MEA, cocamide DEA, cocamide TEA. The amount of non-ionic surfactants used in the composition may vary in a broad range and can be determined by simple routine experiments. Suitable are, for example 5-30wt-% of surfactant in the composition. Such amounts ensure a good foam height. For increasing foam height, poloxamers can be added.

Preferred zwitter-ionic surfactants are selected from the group consisting of Cetyl Betaine, Cocamidopropyl betaine, Dodecyl betaine, Betaine oxides, etc. The amount of zwitter-ionic surfactants used in the composition may vary in a broad range and can be determined by simple routine experiments. Suitable are, for example 1 - 30wt.-%, preferably 5 - 30wt.-% of surfactant in the composition. Such amounts ensure a good foam height.

Preferred cationic surfactants are selected from the group consisting of quaternary ammonium salts Benzalkonium chloride, Cetylpyridinium chloride, Cetylpyridinium bromide. The amount of cationic surfactants used in the composition may vary in a broad range and can be determined by simple routine experiments. However, cationic surfactants are less preferred since they significantly lose their surfactant properties in alkaline pH.

Preferred anionic surfactants are selected from the group consisting of Alkali metal and Ammonium salts of Lauryl, laureth, Lauroyl Sarcosinate and ether carboxylates. The amount of anionic surfactants used in the composition may vary in a broad range and can be determined by simple routine experiments. Suitable are, for example 0.1 - 10wt.-%, preferably 0.1 - 5 wt.-% of surfactant in the composition. However, anionic surfactants are less preferred since they are reported as skin/soft tissue irritants, except for ether carboxylates which are very mild and stable with oxidizing agents over a wide range of pH.

Flavouring agents: One or more flavouring agents (which shall also include sweeteners and fragrances) may be incorporated in said first and / or said second composition. Suitable flavouring agents, sweeteners and fragrances are known in the field. Sweeteners are preferably selected from the group consisting of xylitol, Potassium Acesulfame, Sodium Saccaharin, Aspartame, Levulose Thaumatine, D-Tryptophan, Dihydrochalcones, Cyclamate, and Dextrose. Suitable are, for example 0.05-10wt-% of sweetener in the composition. Flavouring agents are preferably selected from the group consisting of Peppermint Oil, Spearmint Oil, Orange Oil, Anise Oil, Wintergreen Oil, Birch Oil, Freshmint flavour, Herbal Flavor, Lemon oil, Green Apple flavour, Eucalyptol. Suitable are, for example 0.01-2wt-% of flavouring agent in the composition.

Nutrients: One or more nutrients may be incorporated in said first and / or said second composition. Suitable nutrients are known in the field and may be selected from the group of Ascorbic Acid (Vitamin C) and its Alkali- or Alkali earth metal salts, Tocopherol (Vitamin E) and its acetate precursors, Phyloquinone (Vitamin K), Vitamin B (B1, B5, B6, B12), Vitamin A and its Retinoid precursors, Vitamin P, Vitamin H.

Preservatives: One or more preservatives may be incorporated in said first and / or said second composition. Suitable preservatives are known in the field and may be selected from the group of naturally occurring preservatives, conventional preservatives, bleaching agents.

Preferred preservatives include naturally occurring preservatives, in particular Xylitol, Thymol, Eucalyptol, Euganol, Oil of Colve, Sweet Birch Oil, Wintergreen Oil, D-Limonene, Mentha Paprita L oil, Spearmint oil. Such naturally occurring preservatives may be employed in a wide range, suitable are, for example, 0.1 - 8 wt-%.

Further preferred preservatives include conventional preservatives, in particular Methyl Paraben, Ethyl Paraben, Propyl Paraben, Domiphene Bromide, Salicylanide, Zinc ions, Stannous ions, Octenidine, Delmopione, Octapinol. Such conventional preservatives may be employed in a wide range, suitable are, for example, 0.1 - 2 wt-%.

Further preferred preservatives may be selected from the group of said bleaching agents. Suitable bleaching agents are selected from the group consisting of Hydrogen Peroxide, PVP-Hydrogen Peroxide, Alkaline Earth Metal Peroxides, Urea Peroxide (Carbamide Peroxide), Alkali Metal Percarbonate, Alkali Metal Persulfate, Alkali Metal Perborate, Alkali Metal Nitrate, Magnesium Monoperthalate, Alkali Metal Hypochlorite, Alkaline Earth Metal Hypochlorite, Alkali Metal Chlorite, Alkaline Earth Metal Chlorite, Alkali Metal Chlorate, Alkaline Earth Metal Chlorate.

A particular preferred preservative is Polyaminopropyl Biguanide, e.g. at a concentration between 0.1 - 4 wt-%, preferably 0.5 - 2.8 wt-%. This preservative is considered a gentle and effective anti-microbial substance that acts against gram negative bacteria, gram positive bacteria, yeast and fungi over a wide range of pH.

In a preferred embodiment, the invention relates to a composition as described herein, wherein one or more bleaching agents are incorporated in either or both of P- and Ca-substrate. Non-limiting examples of such substances include alkaline earth and group 12 in periodic table metal peroxides; alkali, alkali earth and group 12 metal chlorites, chlorates and hypochlorites; alkali, alkali earth and group 12 metal perborates, percarbonates, persulfates, peroxyphosphates, perthalates and oxoborates; alkali, alkali earth and group 12 metal nitrates; hydrogen peroxide, urea/carbamide peroxide, benzoyl perxode, PVP-hydrogen peroxides, etc. Any alkali metal type said bleaching agents, if present in composition, should preferably incorporated into P-susbstrate; since they react with calcium, they are not suitable to be incorporated into Ca-substrate. Alkali earth and group 12 metal type said bleaching agents, such as CaO2, ZnO2, Ca(OCl)Cl, MgO2, etc., if present in composition, should preferably be added to Ca-substrate; since they react with said phosphate ion releasers, they are suitable to be incorporated in P-substrate. If one or a mixture from a group comprising hydrogen peroxide, urea/carbamide peroxide, benzoyl peroxide and PVP-hydrogen peroxides is be present in the composition, it should be preferably incorporated in P-substrate. The use level may vary over a broad range between 0.001 - 40 wt.-%, preferably 0.01 - 8 wt.-%.

Carrier: Suitable carriers include aqueous gelling agents (in case of aqueous compositions), or non-aqueous gelling agents (in case of non-aqueous compositions), and surfactants; said carrier may be incorporated either in one or both substrates. Polawax is a further suitable carrier within the scope of the invention.

Further additives: One or more further additives may be incorporated in said first and / or said second composition. Suitable further additives are known in the field and may be selected from the group of oral isotonic agents (such as Sodium Chloride), enzymes (such as protease(s), lipase(s), Carbohydrase(s), Q10, FAD), colorants, perfumes, polishing agents.

Among the various embodiments and preferences provided above for the components of the present invention, the specific compositions discussed below are considered particularly advantageous; the invention thus particularly relates to these embodiments.

In an advantageous embodiment, the invention relates to an oral care composition as disclosed herein comprises of two complementary compositions ("substrates") of which one is phosphate (P) enriched and other one is calcium (Ca) enriched. In this embodiment, the P-substrate comprises i) one or more phosphate ion releasing compounds, ii) one or more film forming agents iii) one or more acrylate polymer ion releasers and iv) optionally abrasive particles as main ingredients. The Ca-substrate comprises i) one or more calcium ion releasing compounds and ii) optionally anhydrous glycerol as main ingredients. The film forming agents (in particular the methacrylates identified above) are adapted to stipulate rapid formation of a thin, solid,'permeable and easily removable film onto tooth surface. The P-substrate may contain one or more fluoride ion releasers. Either one or both substrates contain one or more silicate ion releasing agents. Preferably, both substrates contain one or more pH buffering agents.

In a further preferred embodiment, the invention relates to a composition as described herein, having a pH of at least 7, preferably between 7 and 10.5, particular preferably between 8 and 9. Such pH may be obtained by adding a pH regulator as known in the art and / or identified herein. Depending on the components used and the aimed pH, it may be necessary to add one or more pH regulators (such as bases or buffer systems) in said first and / or said second composition. Such regulators are known in the field. Non-limiting examples of pH buffer agents include Borate/alkali hydroxide, mixture of various phosphate salts, carbonate/bicarbonate, alkali metal pyrophosphate/phosphoric acid, etc. It is believed that the alkaline pH stipulates the formation of complex adduct between ACP and calcium hydroxide, hypothetically co-catalyzed/guidance by silicate and acrylate polymer ions, to yield the hydroxyl calcium apatite (HCA) with desired physiochemical properties to closely match with those of tooth, which is the main natural constituent of dentin and enamel.

In a further preferred embodiment, the invention relates to a composition as described herein, which is substantially free of water or free of water. Thus, either one or both substrates may be free (or substantially free) of water. In non-aqueous, or substantially non-aqueous, subsystem it is possible, and advantageous, to incorporate an oxygen and / or a chlorine releasing substances. Non-limiting examples of such substances include alkaline earth and group 12 in periodic table metal peroxides; alkali, alkali earth and group 12 metal chlorites, chlorates and hypochlorites; alkali, alkali earth and group 12 metal perborates, percarbonates, persulfates, peroxyphosphate, perthalates and oxoborates; alkali, alkali earth and group 12 metal nitrates; hydrogen peroxide, urea/carbamide peroxide, benzoyl perxode, PVP-hydrogen peroxides, etc. Some specific examples are MgO2, ZnO2, NaClO2, NaClO3, KClO3, KNO3, Na2B2O8H4, Na2S2O8, CaO2, Ca(OCl)2, Ca(OCl)Cl,Ca(ClO3)2, Ca(NO3)2, etc. The bleaching effect of these substances is supported and / or increased when applied with a sonic vibration toothbrush, due to the locally elevated temperature said device produces. If the said metallic bleaching agents, except nitrates, are incorporated, they produce advantageous hydroxyl ions due to hydrolysis reaction. The hydroxyl ions then raise the pH of the medium, penetrate easily to the deeper/inner structure of tooth facilitated by sonic vibration, and stipulate the transformation of less porous ACP grains to HCA.

In a further preferred embodiment, the invention relates to a composition as described herein, in particular a dental cream, having a viscosity in the range of 10000-30000 cps (10-30 Pa*s). These viscosity ranges are considerably lower than the viscosity of currently available ("classic") toothpastes. It is believed that this lower viscosity significantly supports the beneficial effects of the inventive toothpaste: The low viscosity as described herein increases the surface-coverage of the composition when applied, it facilitates the mobility of the ions, it promotes the formation of thin film on teeth surface, and it also makes it more suitable for application by sonic tooth brush. Carrier, binder, thickening agent and surfactants play an important role in modification to the resulting viscosity of the inventive composition. To adjust the viscosity, an appropriate selection of these components may be employed.

In a further preferred embodiment, the invention relates to a composition as described herein, which is a dental cream, in particular a tooth paste or tooth gel. A person skilled in the art is in a position to choose appropriate additives, in particular selected from the lists provided herein, to obtain a suitable dental cream. As indicated above, such dental creams are particular efficient in oral care, such as for oral hygiene, prevention of teeth decay and for whitening of teeth. Such dental cream is particularly efficient when applied by an electrical tooth brush, such as a sonic vibration tooth brush.

In a further preferred embodiment, the invention relates to a composition as described herein, which is an oral solution, in particular, a spray formulation. Such oral formulations are particular useful for easy to use oral hygiene applications.

In a further preferred embodiment, the invention relates to a composition as described herein, which is a dual snap cream. In this context, "dual snap" defines a single-use packaging type containing an amount of the inventive formulations suitable for a single application. Such packages are known for one component creams; in the present case, such package is adapted to contain the first and second component separately from each other until dispensed. Such formulations may be useful for professional care applications.

In a second aspect, the invention relates to a method for manufacturing an oral care composition. An oral care composition as disclosed herein may be prepared by processes that, though not applied hitherto for the new compositions of the present invention where they thus form new processes, are known per se.

Thus, the invention also relates to a method of manufacturing an oral care composition as disclosed herein comprising the steps of i) manufacturing a composition containing all ingredients of the oral care composition as described herein, except the calcium, phosphate and fluoride (if present) ion releaser; ii) dividing the thus obtained composition into two parts; iii) adding one or more calcium ion releaser, optionally in the presence of further additives as defined herein, to said first part; iv) adding one or more phosphate ion releaser and fluoride ion releaser (if present), optionally in the presence of further additives as defined herein, to the other part and v) filling the thus obtained first and second composition into a suitable two compartment device.

More specific examples for the preparation of aqueous oral care compositions and non-aqueous oral care compositions are given in the context of the examples.

In a third aspect, the invention relates to a method of cleaning / whitening the teeth of a subject in need thereof comprising the step of brushing the teeth with an electrical tooth brush by using a dental cream as disclosed herein (e.g. a tooth paste or tooth gel as disclosed herein). Without being bound to theory, it is believed that electrical tooth brushes, sonic toothbrushes in particular, are beneficial due to the effects explained below:
a) Porosity of ACP plays a crucial role in re-mineralizing / mineralizing the subsurface of tooth. Vibration stipulates close-pack structure to a crystal which is born out of a nucleation of the nano-crystallites or grains. Therefore, sonic vibration facilitates formation of less porous ACP, which is chemically more reactive than the more porous ACP, due to its larger surface active sites that readily bind to other chemicals to form complex adducts, such as HCA, Fluoro-apatite (FCA). Furthermore, sonic vibration locally elevates the temperature which accelerates the rate of ion-diffusion to inner structure of the tooth and catalyzes the nucleation of the nano-grains thereby as well as the subsequent formation of complex adducts like HCA and FCA.
b) Local elevation of temperature also assists rather rapid formation of the thin film
c) Sonic vibration toothbrush device offers an excellent means for removal of dental plaque. Therefore, it further assists in reducing the possibility of mineralizing the dental plaque. Furthermore, it easily and efficiently removes the temporary thin film that arrests superficial ACP and dental plaque. Thus, it contributes towards avoiding undesirable tooth-surface mineralization.

Thus, the method disclosed herein is efficient for subsurface bio-mineralization of the dentin and / or enamel by ACP which is less porous when compared with the prior art, which is further facilitated by the mechanical vibration that the electrical (in particular sonic) care toothbrush offers.

In a fourth aspect, the invention relates to a composition as described herein as pharmaceutical. In particular, the composition is useful for the treatment of diseases and / or disorders related to loss of material of the teeth (in particular HCA). Generally, loss of material of the teeth is due to chemical (biochemical) and / or mechanical reasons. Both aspects are addressed by the present invention. Thus, diseases and / or disorders related to the loss of material may be due to aging, caries (carious lesion), and abrasion / erosion. Further, hypersensitivity and discoloration, which are considered a consequence of loss of material, may be treated as well. The invention thus relates to the treatment, prevention and or delay of progression of such disorders or diseases. It was surprisingly found that a constant use / application of the composition as described herein provides a good remedy to these diseases / disorders. The invention thus further relates to the use of a composition as described herein for the treatment of any of the above identified disorders. The invention also relates to the use of a composition as described herein for the manufacture of a pharmaceutical for the treatment of any of the above identified disorders.

The following examples further illustrate the invention without any limitation; examples 6, 10A and 10B do not form part of the invention but represent background art useful for understanding the invention.

General procedure for manufacturing an aqueous oral care composition: A. Disperse the gelling agent in a mixture of water and humectants with moderate agitation; if required, depending on gelling agents, heat the dispersion to facilitate gellation, followed by cooling down to room temperature. B. Add sweetening and colouring agents. C. Add abrasives during vacuum milling and divide the obtained composition into two parts. D. Add the phosphate and fluoride ion releaser and other electrolytes, except any bleaching agent, in dilute form to the first part of the mixture to produce P-substrate. E. Add calcium ion releaser and other electrolytes, except any bleaching agent, in dilute form to the second part of this composition to produce the Ca-substrate. F. Prepare outside an emulsion of surfactant and flavouring and other lipophilic substances and add separately to either any or both of P- and Ca- substrates. G. Add bleaching agent to any or both P- and Ca-substrates depending on the type of compatibility, if any bleaching agent is desired to be present in the composition. H. Finally add the film forming agent, preferably in emulsion form, to either or both substrate (presently we recommend in P-substrate, as discussed in film agent section). I. Fill in the Ca and P substrate into a two-compartment device.

When necessary, P- and Ca- substrate should be manufactured separately following same general procedure, cited above.

General procedure for manufacturing a non-aqueous oral care composition: A. Disperse a non-aqueous gelling agent (i.e. solid hydrophobic surfactant) and amphiphilic surfactant thickener agent into a composition of humectants/solvent containing flavouring, sweetening and / or colouring agents, and heat the obtained suspension at about 60C while constantly stirring. B. Allow the obtained composition to cool down to room temperature while constantly stirring to obtain a gel. C. Add the abrasives during vacuum milling. and divide the composition into two parts. D. Add the phosphate and fluoride ion releaser and other electrolytes to the first part of the mixture to produce P-substrate. E. Add calcium ion releaser and other electrolytes to the second part of this composition to produce the Ca-substrate. F. Add the film forming agent during milling, preferably to P-substrate G. Fill in the Ca and P substrate into a two-compartment device.

The ingredients used are commercially available and / or obtainable according to known procedures; further they fulfil the requirements for use as oral care products.

Example 1: A non-aqueous toothpaste/Gel is manufactured using the following ingredients.

| Ingredients | | | |
|---|---|---|---|
| Ca-substrate | wt.-% | P-Substrate | wt.-% |
| D-Sorbitol | 40 | D-Sorbitol | 40 |
| Anhydrous Glycerol (or 99.5%) | 10 | Anhydrous Glycerol (or 99.5%) | 10 |
| Hydrogenated Castor Oil PEG 400 | 10 | Hydrogenated Castor Oil PEG 400 | 10 |
| Sorbitan monostearate | 2 | Sorbitan monostearate | 2 |
| Polysorbate 20 | 25 | Polysorbate 20 | 25 |
| Brushite | 2 | Acrylates/Ammonium | 4 |
| | | Methacrylate Copolymer | |
| Fumed Silica | 4 | Fumed Silica | 4 |
| Polyaminopropyl Biguanide | 1 | Polyaminopropyl Biguanide | 0.6 |
| Calcium Peroxide | 0.1 | Dipotassium Phosphate | 2 |
| Calcium fumarate | 4.5 | Monosodium Phosphate | 2 |
| Calcium Silicate | 0.5 | Sodium Monfluorophosphate | 0.5 |
| Potassium Acesulfame | 0.5 | Potassium Acesulfame | 0.5 |
| Peppermint Oil | 0.4 | Peppermint Oil | 0.4 |

Example 2: A non-aqueous toothpaste / Gel is manufactured using the following ingredients.

| Ca-substrate | wt.-% | P-Substrate | wt.-% |
|---|---|---|---|
| D-Sorbitol | 20 | D-Sorbitol | 20 |
| Anhydrous Glycerol (or 99.5%) | 20 | Anhydrous Glycerol (or 99.5%) | 20 |
| Hydrogenated Castor Oil PEG 400 | 20 | Hydrogenated Castor Oil PEG 400 | 20 |
| Sorbitan monostearate | 2 | Sorbitan monostearate | 2 |
| Polysorbate 80 | 25 | Polysorbate 80 | 25 |
| Brushite | 1 | Acrylates/Ammonium Methacrylate Copolymer | 4 |
| Fumed Silica | 4 | Fumed Silica | 4 |
| PVM/MA | 1 | Polyaminopropyl Biguanide | 0.6 |
| Polyaminopropyl Biguanide | 1 | Ammonium Phosphate | 1 |
| Calcium Hypochlorite | 0.1 | Potassium Phosphate | 1 |
| Calcium Malate | 2.5 | Sodium Phosphate | 1 |
| Calcium Glycerophosphate | 1 | Monopotassium Phosphate | 1 |
| Calcium lactate gluconate | 1 | Sodium Monfluorophosphate | 0.5 |
| Calcium Silicate | 0.5 | Potassium Acesulfame | 0.5 |
| Potassium Acesulfame Peppermint Oil | 0.5 0.4 | Peppermint Oil | 0.4 |

Example 3: Aqueous toothpaste/Gel with a viscosity of about 20 Pa*s is manufactured using the following ingredients.

| Ca-substrate | wt.-% | P-substrate | wt.-% |
|---|---|---|---|
| Deionized Water | 23 | Deionized Water | 23 |
| Sorbitol | 12.3 | Sorbitol | 12.3 |
| Glycerin | 10 | Glycerin | 10 |
| PEG 600 | 5 | PEG 600 | 5 |
| Sodium carboxymethylcellulose | 0.8 | Sodium carboxymethylcellulose | 0.8 |
| Xanthan Gum | 1 | Xanthan Gum | 1 |
| Polysorbate 20 | 20 | Polysorbate 20 | 20 |
| Hydrated Silica | 5 | Hydrated Silica | 5 |
| Brushite | 4 | PM/MA Copolymer | 4 |
| Sodium Bicarbonate | 5 | Sodium Bicarbonate | 5 |
| Calcium Nitrate | 2 | Tripotassium Phosphate | 1.5 |
| Calcium Glycerophosphate | 4 | Monosodium Phosphate | 1.5 |
| Calcium Silicate | 1 | Bipotassium Phosphate | 1 |
| Sodium Hydroxide | 0.2 | Sodium monofluorophospahte | 0.7 |
| Sodium Chloride | 2 | Acrylates/Ammonium Methacrylate Copolymer | 5 |
| Potassium Acesulfame | 0.5 | Potassium Acesulfame | 0.5 |
| Flavour | 0.5 | Flavour | 0.5 |
| Polyaminopropyl Biguanide | 1.5 | Polyaminopropyl Biguanide | 1.5 |
| TiO2 | 0.1 | PVM/MA | 1.7 |
| ZnO | 0.1 | | |
| Xilitol | 2 | | |

Example 4: Aqueous toothpaste with a viscosity of about 20 Pa*s is manufactured using the following ingredients.

| Ca-substrate | wt.-% | P-substrate | wt.-% |
|---|---|---|---|
| Deionized Water | 35 | Deionized Water | 35 |
| Sorbitol(70%) | 40 | Sorbitol(70%) | 40 |
| Glycerol | 1.83 | Glycerol | 1 |
| Carbopol 947P NF polymer | 0.7 | Carbopol 947P NF polymer | 0.7 |
| Xanthan Gum | 0.6 | Xanthan Gum | 0.6 |
| Diagum CS Refined Cassia | 0.07 | Diagum CS Refined Cassia | 0.07 |
| Sodium Lauryl Sarcosinate | 1.5 | Sodium Lauryl Sarcosinate | 1.5 |
| Hydrated Silica | 4 | Hydrated Silica | 4 |
| Brushite | 4 | PM/MA Copolymer | 5 |
| Calcium Carbonate | 2 | Acrylates/Ammonium Methacrylate Copolymer | 5 |
| Sodium Bicarbonate | 2 | PVM/MA Copolymer | 0.3 |
| Calcium Nitrate | 2 | Disodium Phosphate | 1.5 |
| Calcium propionate | 2 | Monpotassium Phosphate | 1.5 |
| Calcium Silicate | 1 | Trisodium Phosphate | 1 |
| Sodium Hydroxide | 0.2 | Sodium Monofluorophosphate | 0.63 |
| Sodium Chloride | 1 | Sodium Hydroxide | 0.2 |
| Potassium Acesulfame | 0.5 | Potassium Acesulfame | 0.5 |
| Flavour | 0.5 | Flavour | 0.5 |
| Polyaminopropyl Biguanide | 1 | Polyaminopropyl Biguanide | 1 |
| TiO2 | 0.1 | | |

Example 5: Aqueous Tooth-Lotion, suitable for dual snap use, is manufactured using the following ingredients.

| Ca-substrate | wt.-% | P-substrate | wt.-% |
|---|---|---|---|
| Deionized Water | 84.3 | Deionized Water | 75.7 |
| Xanthan Gum | 1 | Xanthan Gum | 1 |
| Calcium Nitrate | 2 | Dipotassium Phosphate | 2 |
| Calcium Malate Citrate | 2 | Monosodium Phosphate | 2 |
| Calcium Silicate | 1 | Sodium Monofluorophosphate | 0.6 |
| Glycerine | 5 | Glycerine | 2 |
| Potassium Acesulfame | 0.5 | Potassium Acesulfame | 0.5 |
| Flavor | 0.5 | Flavor | 0.5 |
| Polyaminopropyl Biguanide | 1.7 | Polyaminopropyl Biguanide | 1.7 |
| Colloidal Silica | 1.5 | PM/MA Copolymer | 1.5 |
| PVM/MA Copolymer | 0.5 | PVM/MA Copolymer | 0.5 |
| | | Acrylates/Ammonium Methacrylate Copolymer | 5 |
| | | Polysorbate 80 | 5 |
| | | Sodium Chloride | 2 |

Example 6: Aqueous tooth re-mineralizing oral solution, suitable to dual-chamber device, is manufactured using the following ingredients.

| Ca-substrate | wt.-% | P-Substrate | wt.-% |
|---|---|---|---|
| Demineralized Water | 98 | Deminaralized Water | 68.8 |
| Calcium Nitrate | 0.5 | Polysorbate 20 | 20 |
| Calcium Propionate | 0.8 | Bipotassium Phosphate | 0.5 |
| Potassium Acesulfame | 0.7 | Bisodium Phosphate | 0.2 |
| | | Monosodium Phosphate | 0.1 |
| | | Monopotassium Phosphate | 0.1 |
| | | Tripotassium Phosphate | 0.1 |
| | | Sodium Monofluorophosphate | 0.5 |
| | | Acrylates/Ammonium Methacrylate Copolymer | 5 |
| | | Polyaminopropyl Biguanide | 1.5 |
| | | Potassium Acesulfame | 0.7 |
| | | Sodium Chloride | 1 |
| | | Peppermint Oil | 0.5 |
| | | Birch Oil | 0.5 |
| | | Eucalyptol Oil | 0.5 |

example 7 Aqueous toothpastes/Gels are manufactured using the following ingredients

### example 7.1

| Ca-Substrate | conc. (%) | P-Substrate | conc. (%) |
|---|---|---|---|
| Water | 30.38 | Water | 30.38 |
| D-Sorbitol | 43 | D-Sorbitol | 43 |
| Carbopol 980 | 0.75 | Carbopol 980 | 0.75 |
| Sodium carboxymethylcellulose | 0.25 | Sodium carboxymethylcellulose | 0.25 |
| Xanthan Gum | 0.50 | Xanthan Gum | 0.50 |
| Zeodent 165 | 5 | Zeodent 165 | 5 |
| Zeodent 115 | 2 | Zeodent 115 | 2 |
| PEG 400 | 10 | PEG 400 | 10 |
| Sodium lauryl sarcosinate | 1.5 | Sodium lauryl sarcosinate | 1.5 |
| NaOH | 0.2 | NaOH | 0.2 |
| Calcium Nitrate | 1.50 | Potassium Phosphate | 1.50 |
| Calcium Propionate | 1 | Monosodium Phosphate | 1 |
| Calcium Silicate | 0.1 | Sodium Monofluorophosphate | 0.14 |
| Polyaminopropyl Biguanide | 1 | Polyaminopropyl Biguanide | 1 |
| Potassium Acesulfame | 0.5 | Potassium Acesulfame | 0.5 |
| Flavour | 0.5 | Flavour | 0.5 |
| NaCl | 0.5 | Poly(methyl vinyl ether-alt-maleic anhydride), cross-linked with 1,9-decadiene | 0.35 |
| Polysorbate 20 | q.s. | Poly(methyl vinyl ether-alt-maleic acid) | 0.50 |
| | | Poly(1-vinylpyrrolidone-co-vinyl acetate) | 0.25 |
| | | Polysorbate 20 | q.s. |

### example 7.2

| Ca-Substrate | conc. (%) | P-Substrate | |
|---|---|---|---|
| Water | 70 | Water | 70 |
| Carbopol 980 | 0.9 | Carbopol 980 | 0.9 |
| Sodium carboxymethylcellulose | 0.25 | Sodium carboxymethylcellulose | 0.25 |
| Xanthan Gum | 0.50 | Xanthan Gum | 0.50 |
| Zeodent 165 | 4 | Zeodent 165 | 4 |
| Zeodent 115 | 4 | Zeodent 115 | 4 |
| Calcium Carbonate | 1 | Sodium biarbonate | 1 |
| PEG 400 | 10.50 | PEG 400 | 10.50 |
| Sodium lauryl sarcosinate | 1.5 | Sodium lauryl sarcosinate | 1.5 |
| NaOH | 0.2 | NaOH | 0.2 |
| Calcium propionate | 1.50 | Monosodium Phosphate | 1.50 |
| Calcium Nitrate | 0.50 | Bipotassium Phosphate | 0.50 |
| Calcium Silicate | 0.1 | Sodium Monofluorophosphate | 0.12 |
| Potassium Acesulfame | 0.5 | Potassium Acesulfame | 0.5 |
| Flavour | 0.5 | Flavour | 0.5 |
| NaCl | 0.5 | NaCl | 0.5 |
| Polyaminopropyl Biguanide | 1 | Polyaminopropyl Biguanide | 1 |
| TiO2 | 0.1 | TiO2 | 0.1 |
| ZnO | 0.1 | ZnO | 0.1 |
| Polysorbate 20 | q.s. | Polysorbate 20 | q.s. |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |

### example 7.3

| Ca-Substrate | conc. (%) | P-Substrate | conc. (%) |
|---|---|---|---|
| Water | 70 | Water | 70 |
| Carbopol 980 | 0.9 | Carbopol 980 | 0.9 |
| Sodium carboxymethylcellulose | 0.25 | Sodium carboxymethylcellulose | 0.25 |
| Xanthan Gum | 0.50 | Xanthan Gum | 0.50 |
| Zeodent 165 | 4 | Zeodent 165 | 4 |
| Zeodent 115 | 4 | Zeodent 115 | 4 |
| Calcium Carbonate | 2 | Sodium biarbonate | 1 |
| PEG 400 | 10.50 | PEG 400 | 10.50 |
| Sodium lauryl sarcosinate | 1.5 | Sodium lauryl sarcosinate | 1.5 |
| NaOH | 0.2 | NaOH | 0.2 |
| Calcium propionate | 1.50 | Monosodium Phosphate | 0.50 |
| Calcium Nitrate | 0.50 | Monopotassium Phosphate | 0.50 |
| Calcium Silicate | 0.1 | Bipotassium Phosphate | 0.50 |
| Potassium Acesulfame | 0.5 | Sodium Phosphate | 0.50 |
| Flavour | 0.5 | Sodium Monofluorophosphate | 0.12 |
| NaCl | 0.5 | Potassium Acesulfame | 0.5 |
| TiO2 | 0.1 | Flavour | 0.5 |
| Sodium Chlorite | 0.3 | NaCl | 0.5 |
| Polysorbate 20 | q.s. | Polyaminopropyl Biguanide | 1 |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Sodium Ascorbate | 0.20 |
| | | Polysorbate 20 | q.s. |

example 8 Non-aqueous toothpastes/Gels are manufactured using the following ingredients

### example 8.1

| Ca-Substrate | wt.-% | P-Substrate | wt.-% |
|---|---|---|---|
| Anhydrous Glycerine (or 99.5%) | 70 | Anhydrous Glycerine (or 99.5%) | 70 |
| Carbopol 980 | 2.0 | Carbopol 980 | 2.0 |
| Xanthan Gum | 0.50 | Xanthan Gum | 0.50 |
| Zeodent 165 | 4 | Zeodent 165 | 4 |
| Zeodent 115 | 4 | Zeodent 115 | 4 |
| PEG 400 | 10.95 | PEG 400 | 10 |
| Sodium lauryl sarcosinate | 1.5 | Sodium lauryl sarcosinate | 1.5 |
| NaOH | 0.3 | NaOH | 0.3 |
| Calcium propionate | 1.5 | Sodium Phosphate | 1 |
| Calcium Nitrate | 0.5 | Potassium Phosphate | 0.5 |
| Calcium Silicate | 0.1 | Monosodium Phosphate | 0.5 |
| Potassium Acesulfame | 0.5 | Potassium Acesulfame | 0.5 |
| Peppermint Oil | 0.5 | Peppermint Oil | 0.5 |
| Birch Oil | 0.1 | Birch Oil | 0.1 |
| Eucalyptol Oil | 0.05 | Eucalyptol Oil | 0.05 |
| NaCl | 0.5 | NaCl | 0.5 |
| Sodium Bicarbonate | 2 | Sodium Monofluorophosphate | 0.12 |
| Polyaminopropyl Biguanide | 1 | Polyaminopropyl Biguanide | 1 |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Sodium Ascorbate | 0.25 |
| | | Polysorbate 20 | q.s. |

### example 8.2

| Ca-Substrate | wt.-% | P-Substrate | wt.-% |
|---|---|---|---|
| Anhydrous Glycerine (or 99.5%) | 70 | Anhydrous Glycerine (or 99.5%) | 70 |
| Carbopol 980 | 2.0 | Carbopol 980 | 2.0 |
| Xanthan Gum | 0.50 | Xanthan Gum | 0.50 |
| Zeodent 165 | 4 | Zeodent 165 | 4 |
| Zeodent 115 | 4 | Zeodent 115 | 4 |
| PEG 400 | 11 | PEG 400 | 11 |
| Sodium lauryl sarcosinate | 1.5 | Sodium lauryl sarcosinate | 1.5 |
| NaOH | 0.3 | NaOH | 0.3 |
| Calcium propionate | 1.5 | Sodium Phosphate | 0.5 |
| Calcium Nitrate | 0.5 | Monopotassium Phosphate | 0.5 |
| Calcium Silicate | 0.1 | Bipotassium Phosphate | 1 |
| Potassium Acesulfame | 0.5 | Sodium Monofluorophosphate | 0.12 |
| Peppermint Oil | 0.5 | Potassium Acesulfame | 0.5 |
| Birch Oil | 0.1 | Peppermint Oil | 0.5 |
| Eucalyptol Oil | 0.05 | Birch Oil | 0.1 |
| NaCl | 0.5 | Eucalyptol Oil | 0.05 |
| Sodium Chlorite | 0.3 | NaCl | 0.5 |
| Polysorbate 20 | 1 | Polyaminopropyl Biguanide | 1 |
| Calcium Carbonate | q.s. | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Polysorbate 20 | q.s. |

### example 8.3

| Ca-Substrate | wt.-% | P-Substrate | wt.-% |
|---|---|---|---|
| Anhydrous Glycerine (or 99.5%) | 71 | Anhydrous Glycerine (or 99.5%) | 71 |
| Carbopol 980 | 2.0 | Carbopol 980 | 2.0 |
| Xanthan Gum | 0.50 | Xanthan Gum | 0.50 |
| Zeodent 165 | 4 | Zeodent 165 | 4 |
| Zeodent 115 | 4 | Zeodent 115 | 4 |
| PEG 400 | 10 | PEG 400 | 10 |
| Sodium lauryl sarcosinate | 1.5 | Sodium lauryl sarcosinate | 1.5 |
| NaOH | 0.3 | NaOH | 0.3 |
| Calcium propionate | 1.5 | Sodium Phosphate | 0.5 |
| Calcium Nitrate | 0.5 | Monopotassium Phosphate | 0.5 |
| Calcium Silicate | 0.1 | Bisodium Phosphate | 0.5 |
| Potassium Acesulfame | 0.5 | Tripotassium Phosphate | 0.5 |
| Peppermint Oil | 0.5 | Sodium Monofluorophosphate | 0.12 |
| Birch Oil | 0.1 | Potassium Acesulfame | 0.5 |
| Eucalyptol Oil | 0.05 | Peppermint Oil | 0.5 |
| NaCl | 0.5 | Birch Oil | 0.1 |
| Metal Peroxide | 0.5 | Eucalyptol Oil | 0.05 |
| Polysorbate 20 | 1 | NaCl | 0.25 |
| Calcium Carbonate | q.s. | Polyaminopropyl Biguanide | 1 |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Sodium Ascorbate | 0.25 |
| | | Polysorbate 20 | q.s. |

Example 9: An aqueous Tooth-Lotion, suitable for dual snap use, is manufactured according to the general procedure using the following ingredients.

### example 9A

| Ca-Substrate | wt.-% | P-substrate | wt.-% |
|---|---|---|---|
| Deionized Water | 35 | Deionized Water | 35.0 |
| Sorbitol | 45.95 | Sorbitol | 43.88 |
| Carbopol 980 | 0.45 | Carbopol 980 | 0.45 |
| Xanthan Gum | 0.50 | Xanthan Gum | 0.50 |
| Glycerine | 5 | Glycerine | 5 |
| PEG 400 | 2.5 | PEG 400 | 2.5 |
| Zeodent 165 | 2.5 | Zeodent 165 | 2.5 |
| Zeodent 115 | 2.5 | Zeodent 115 | 2.5 |
| NaOH | 0.30 | NaOH | 0.3 |
| Sodium lauryl sarcosinate | 1.5 | Sodium lauryl sarcosinate | 1.5 |
| Calcium Propionate | 1.5 | Dipotassium Phosphate | 1.5 |
| Calcium Nitrate | 0.5 | Monosodium Phosphate | 0.5 |
| Calcium Silicate | 0.1 | Sodium Monofluorophosphate | 0.12 |
| Potassium Acesulfame | 0.5 | Potassium Acesulfame | 0.5 |
| Flavor | 0.3 | Flavor | 0.3 |
| Polyaminopropyl Biguanide | 1.0 | Polyaminopropyl Biguanide | 1 |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Sodium Chloride | 0.25 |
| | | Sodium Ascorbate | 0.20 |
| | | Tocopherol Acetate | 0.20 |
| | | Papain | 0.20 |

### Example 9B:

| Ca-substrate | wt.-% | P-substrate | wt.-% |
|---|---|---|---|
| Deionized Water | 73.35 | Deionized Water | 71.03 |
| Sodium carboxymethylcellulose | 0.25 | Sodium carboxymethylcellulose | 0.25 |
| Carbopol 980 | 0.75 | Carbopol 980 | 0.75 |
| Xanthan Gum | 0.50 | Xanthan Gum | 0.50 |
| Polysorbate 20 | 5 | Polysorbate 20 | 5 |
| PEG 400 | 8 | PEG 400 | 8 |
| Zeodent 165 | 4 | Zeodent 165 | 4 |
| Zeodent 115 | 3 | Zeodent 115 | 3 |
| NaOH | 0.30 | NaOH | 0.3 |
| Sodium lauryl sarcosinate | 1.5 | Sodium lauryl sarcosinate | 1.5 |
| Calcium Propionate | 1 | Dipotassium Phosphate | 1 |
| Calcium Nitrate | 1 | Monosodium Phosphate | 1 |
| Calcium Silicate | 0.1 | Sodium Monofluorophosphate | 0.12 |
| Potassium Acesulfame | 0.5 | Potassium Acesulfame | 0.5 |
| Flavor | 0.5 | Flavor | 0.3 |
| Sodium Chlorite | 0.25 | Polyaminopropyl Biguanide | 1 |
| | | Poly(methyl vinyl ether -alt-maleic anhydride), cross-linked with 1,9-decadiene | 0.35 |
| | | Poly(methyl vinyl ether-alt-maleic acid) | 0.50 |
| | | Poly(1-vinylpyrrolidone-co-vinyl acetate) | 0.25 |
| | | Sodium Chloride | 0.25 |
| | | Tocopherol Acetate | 0.20 |
| | | Papain | 0.20 |

### Example 9C:

| Ca-Substrate | wt.-% | P-substrate | wt.-% |
|---|---|---|---|
| Deionized Water | 71.35 | Deionized Water | 68.53 |
| Hydroxypropyle Cellulose | 2.25 | Hydroxypropyle Cellulose | 2.25 |
| Xanthan Gum | 1.25 | Xanthan Gum | 1.25 |
| Polysorbate 20 | 5 | Polysorbate 20 | 5 |
| PEG 400 | 8 | PEG 400 | 8 |
| Zeodent 165 | 4 | Zeodent 165 | 4 |
| Zeodent 115 | 3 | Zeodent 115 | 3 |
| NaOH | 0.30 | NaOH | 0.3 |
| Sodium lauryl sarcosinate | 1.5 | Sodium lauryl sarcosinate | 1.5 |
| Calcium Propionate | 1 | Dipotassium Phosphate | 1 |
| Calcium Nitrate | 1 | Monosodium Phosphate | 1 |
| Calcium Silicate | 0.1 | Sodium Monofluorophosphate | 0.12 |
| Potassium Acesulfame | 0.5 | Potassium Acesulfame | 0.5 |
| Flavor | 0.5 | Flavor | 0.3 |
| Sodium Chlorite | 0.25 | Polyaminopropyl Biguanide | 1 |
| | | Poly(methyl vinyl ether -alt-maleic anhydride), | 0.35 |
| | | cross-linked with 1,9-decadiene | |
| | | Poly(methyl vinyl ether -alt-maleic acid) | 0.50 |
| | | Poly(1-vinylpyrrolidone -co-vinyl acetate) | 0.50 |
| | | Sodium Chloride | 0.50 |
| | | Sodium Ascorbate | 0.20 |
| | | Papain | 0.20 |

Example 10: An aqueous tooth remineralizing oral solution, suitable to dual-chamber device, is manufactured using the following ingredients.

### Example 10A:

| Ca-Substrate | wt.-% | P-Substrate | wt.-% |
|---|---|---|---|
| Demineralized Water | 77.7 | Deminaralized Water | 67.83 |
| Calcium Nitrate | 0.5 | Polysorbate 20 | 25 |
| Calcium Propionate | 0.8 | Bipotassium Phosphate | 0.5 |
| Polysorbate 20 | 20 | Bisodium Phosphate | 0.2 |
| Flavor | 0.3 | Monosodium Phosphate | 0.1 |
| Potassium Acesulfame | 0.7 | Monopotassium Phosphate | 0.1 |
| | | Tripotassium Phosphate | 0.1 |
| | | Sodium Monofluorophosphate | 0.12 |
| | | Poly(methyl vinyl ether -alt-maleic anhydride), cross-linked with 1,9-decadiene | 0.35 |
| | | Poly(methyl vinyl ether -alt-maleic acid) | 0.50 |
| | | Sodium Ascorbate | 0.25 |
| | | Tocopherol Acetate | 0.25 |
| | | Polyaminopropyl Biguanide | 1.5 |
| | | Potassium Acesulfame | 0.7 |
| | | Sodium Chloride | 1 |
| | | Peppermint Oil | 0.5 |
| | | Birch Oil | 0.5 |
| | | Eucalyptol Oil | 0.5 |

### Example 10B:

| Ca-Substrate | wt.-% | P-Substrate | wt.-% |
|---|---|---|---|
| Demineralized Water | 96.7 | Deminaralized Water | 67.40 |
| Hydroxypropyl Cellulose | 0.75 | Carbopol 980 | 0.13 |
| NaOH | 0.3 | Xanthan Gum | 0.20 |
| Sodium Chlorite | 0.25 | NaOH | 0.10 |
| Calcium Nitrate | 0.5 | Polysorbate 20 | 25 |
| Calcium Propionate | 0.8 | Bipotassium Phosphate | 0.5 |
| Potassium Acesulfame | 0.7 | Bisodium Phosphate | 0.2 |
| | | Monosodium Phosphate | 0.1 |
| | | Monopotassium Phosphate | 0.1 |
| | | Tripotassium Phosphate | 0.1 |
| | | Sodium Monofluorophosphate | 0.12 |
| | | Poly(methyl vinyl ether -alt-maleic anhydride), cross-linked with 1,9-decadiene | 0.35 |
| | | Poly(methyl vinyl ether-alt-maleic acid) | 0.50 |
| | | Sodium Ascorbate | 0.25 |
| | | Tocopherol Acetate | 0.25 |
| | | Polyaminopropyl Biguanide | 1.5 |
| | | Potassium Acesulfame | 0.7 |
| | | Sodium Chloride | 1 |
| | | Peppermint Oil | 0.5 |
| | | Birch Oil | 0.5 |
| | | Eucalyptol Oil | 0.5 |

Example 11: A clinical tooth remineralizing oral gel, suitable to dual-chamber device, is manufactured using the following ingredients. These examples contain comparatively higher amounts of bio-mineralizing agents and bleaching agents. These compositions are therefore particular suitable for professionals, such as dentists or people otherwise educated in the field.

### Example 11A:

| Ca-Substrate | wt.-% | P-Substrate | wt.-% |
|---|---|---|---|
| Propylene Glycol | 30 | Propylene Glycol | 30 |
| Glycerin | 13.5 | Glycerin | 18 |
| Hydroxypropyl Cellulose | 2.2 | Hydroxypropyl Cellulose | 2.2 |
| NaOH | 0.3 | NaOH | 0.30 |
| Fumed Silica (Carbosil) | 4.8 | Carbosil | 4 |
| Sodium Chlorite | 20 | Pluronic L64 | 20.85 |
| Calcium Nitrate | 15 | Bipotassium Phosphate | 2 |
| Calcium Propionate | 4 | Bisodium Phosphate | 2 |
| Calcium Silicate | 0.5 | Monosodium Phosphate | 2 |
| Polawax | 9 | Monopotassium Phosphate | 2 |
| Potassium Acesulfame | 0.7 | Tripotassium Phosphate | 11 |
| | | Sodium Monofluorophos-phate | 0.8 |
| | | Poly(methyl vinyl ether -alt-maleic anhydride), cross-linked with 1,9-decadiene | 0.35 |
| | | Poly(methyl vinyl ether-alt-maleic acid) | 0.50 |
| | | Sodium Ascorbate | 0.25 |
| | | Tocopherol Acetate | 0.25 |
| | | Polyaminopropyl Biguanide | 1 |
| | | Potassium Acesulfame | 0.5 |
| | | Sodium Chloride | 1 |
| | | Peppermint Oil | 0.4 |
| | | Birch Oil | 0.2 |
| | | Eugenol | 0.2 |
| | | Eucalyptol Oil | 0.2 |

### Example 11B:

| Ca-Substrate | wt.-% | P-Substrate | wt.-% |
|---|---|---|---|
| Deionized Water | 52 | Deionized Water | 47.45 |
| Carbopol 980 | 0.40 | Carbopol 980 | 0.40 |
| Hydroxypropyl Cellulose | 2.2 | Hydroxypropyl Cellulose | 2.2 |
| NaOH | 0.2 | NaOH | 0.20 |
| Fumed Silica (Carbosil) | 4 | Carbosil | 4 |
| Sodium Chlorite | 20 | Pluronic L64 | 20 |
| Calcium Nitrate | 15 | Bipotassium Phosphate | 2 |
| Calcium Propionate | 5 | Bisodium Phosphate | 3 |
| Calcium Silicate | 0.5 | Monosodium Phosphate | 4 |
| Potassium Acesulfame | 0.7 | Monopotassium Phosphate | 6 |
| | | Tripotassium Phosphate | 5 |
| | | Sodium Monofluorophosphate | 0.8 |
| | | Poly(methyl vinyl ether-alt-maleic anhydride), cross-linked with 1,9-decadiene | 0.35 |
| | | Poly(methyl vinyl ether-alt-maleic acid) | 0.50 |
| | | Sodium Ascorbate | 0.25 |
| | | Tocopherol Acetate | 0.25 |
| | | Polyaminopropyl Biguanide | 1 |
| | | Potassium Acesulfame | 0.5 |
| | | Sodium Chloride | 1 |
| | | Peppermint Oil | 0.5 |
| | | Birch Oil | 0.2 |
| | | Eugenol | 0.2 |
| | | Eucalyptol Oil | 0.2 |

Example 12 An aqueous personal care dental cream / toothpaste, suitable to dual-chamber device, is manufactured using the following ingredients.

### Example 12.1

| Ca-Substrate | wt.-% | P-Substrate | wt.-% |
|---|---|---|---|
| Water | 20 | Water | 19 |
| Sorbitol (70%) | 40.647 | Sorbitol (70%) | 40.62 |
| Sident 8 | 10 | Sident 8 | 10 |
| Sident 22S | 5.5 | Sident 22S | 5.5 |
| Xanthan gum | 0.37 | Xanthan gum | 0.37 |
| Natrosol 250 MRB | 0.75 | Natrosol 250 MRB | 0.75 |
| Glycerin (99.5%) | 9 | Glycerin (99.5%) | 9 |
| Calcium Nitrate | 2 | Tripotassium Phosphate | 1.5 |
| Calcium Propionate | 2 | Disodium Phosphate | 1.5 |
| Calcium Silicate | 1 | Monosodium Phosphate | 0.5 |
| Sodium Hydroxide | 0.3 | Sodium Monofluorophosphate | 1 |
| Cocamidopropyl Betain (30%) | 4.5 | Acrylates/Ammonium Methacrylate Copolymer | 5 |
| Sodium Saccharin | 0.083 | Boric acid | 1.36 |
| Peppermint oil | 1.35 | Sodium Hydroxide | 1.5 |
| Polyaminopropyl Biguanide | 1.5 | Sodium Polyacrylate | 0.4 |
| TiO2 | 1 | Sodium Chloride | 1 |
| | | Polyaminopropyl Biguanide | 1 |

### Example 12.2

| Ca-Substrate | wt.-% | P-Substrate | wt.-% |
|---|---|---|---|
| Water | 21.067 | Water | 19.457 |
| Sorbitol (70%) | 41 | Sorbitol (70%) | 36 |
| Syloid AL-1FP | 2 | Syloid AL-1FP | 2 |
| Syloblanc 34 | 9 | Syloblanc 34 | 9 |
| Xanthan gum | 0.3 | Xanthan gum | 0.3 |
| Natrosol 250 HRB | 0.85 | Natrosol 250 HRB | 0.85 |
| Glycerin (99.5%) | 9 | Glycerin (99.5%) | 9 |
| Calcium Fumarate | 2 | Dipotassium hydrogen phosphate | 3.5 |
| Calcium Propionate | 2 | Monopotassium dihydrogen phosphate | 0.03 |
| Calcium Silicate | 1 | Monosodium dihydrogen phosphate | 0.05 |
| Calcium Hypochlorite | 1 | Disodium hydrogen phosphate | 1 |
| Sodium Hydroxide | 0.3 | Acrylates/Ammonium | 4.5 |
| | | Methacrylate Copolymer | |
| Cocamine Oxide (30%) | 5 | Boric acid | 2.73 |
| Sodium Saccharin | 0.083 | Sodium Hydroxide | 2.5 |
| Flavor | 1 | Sodium monofluorophosphate | 1 |
| Brushite | 3.3 | Carbopol Aqua SF-1 | 1 |
| Wintergreen oil | 0.1 | Isopropanol | 6 |
| TiO2 | 1 | Sodium Saccharin | 0.083 |
| | | Sodium Chlorite | 1 |

### Example 12.3:

| Ca-Substrate | wt.-% | P-Substrate | wt.-% |
|---|---|---|---|
| Water | 20.167 | Water | 19.457 |
| Sorbitol (70%) | 41 | Sorbitol (70%) | 36.13 |
| Syloid AL-1FP | 2 | Syloid AL-1FP | 2 |
| Syloblanc 34 | 9 | Syloblanc 34 | 6.5 |
| Walen Gum | 0.3 | Optigel | 3 |
| Natrosol 250 HRB | 0.85 | Natrosol 250 HRB | 1 |
| Glycerin (99.5%) | 9 | Glycerin (99.5%) | 9 |
| Calcium Propionate | 3 | Dipotassium hydrogen phosphate | 0.5 |
| Calcium Hydroxide | 0.5 | Trisodium phosphate | 1 |
| Calcium Silicate | 1.5 | Monosodium dihydrogen phosphate | 0.1 |
| Calcium Hypochlorite | 1 | Disodium hydrogen phosphate | 2.5 |
| Magensium aluminum silicate colloidal | 2 | Acrylates/Ammonium Methacrylate Copolymer | 5 |
| Cocamine Oxide (30%) | 4.5 | Boric acid | 2.73 |
| Sodium Saccharin | 0.083 | Sodium Hydroxide | 1.5 |
| Flavor | 1 | Sodium Fluoride | 1 |
| Sodium Chloride | 0.5 | Carbopol Aqua SF-1 | 2 |
| Brushite | 2.5 | Cocamine oxide (30%) | 4.5 |
| Wintergreen oil | 0.1 | Sodium Saccharin | 0.083 |
| TiO2 | 1 | Flavor | 1 |
| | | Sodium Chlorite | 1 |

Example 13: A low Aqueous personal care dental cream / toothpaste, suitable for a dual-chamber device, is manufactured using the following ingredients.

### Example 13.1:

| Ca-Substrate | wt.-% | P-Substrate | wt.-% |
|---|---|---|---|
| Water | 10 | Water | 10 |
| Polyisodecene | 40 | Polyisodecene | 38 |
| Syloid AL-1FP | 2 | Syloid AL-1FP | 2 |
| Syloblanc 34 | 6 | Syloblanc 34 | 6 |
| Thixogel | 8 | Thixogel | 8 |
| Glyceryl Behenate/Eicosadioate | 4 | Glyceryl Behenate/ Eicosadioate | 4 |
| Natrosol 250 HRB | 0.5 | Polyglyceryl-10 Behenate/Eicosadioate | 2 |
| Glycerin (99.5%) | 4.817 | Natrosol 250 HRB | 0.5 |
| Calcium Propionate | 5 | Disodium hydrogen phosphate | 4 |
| Calcium Hydroxide | 0.5 | Monosodium dihydrogen phosphate | 0.1 |
| Calcium Silicate | 1.5 | Trisodium Phospshate | 1 |
| Calcium Hypochlorite | 2 | Sodium Fluoride | 1 |
| Magensium aluminum silicate colloidal | 3 | Carbopol Aqua SF-1 | 2 |
| Cocamidopropyl Betain (30%) | 4.5 | Cocamine Oxide (30%) | 4.5 |
| Sodium Saccharin | 0.083 | Sodium Chlorite | 1 |
| Flavor | 1.5 | Sodium Saccharin | 0.083 |
| Sodium Chloride | 0.5 | Flavor | 0.817 |
| Brushite | 4 | Acrylates/Ammonium Methacrylate Copolymer | 5 |
| Calcium Carbonate | 1 | Propylene Glycol | 10 |
| Wintergreen oil | 0.1 | | |
| TiO2 | 1 | | |

### Example 13.2:

| Ca-Substrate | wt.-% | P-Substrate | wt.-% |
|---|---|---|---|
| Water | 26 | Water | 10 |
| Polyisodecene | 27 | Polyisodecene | 40 |
| Syloid AL-1FP | 2 | Syloid AL-1FP | 2 |
| Syloblanc 34 | 6.5 | Syloblanc 34 | 6 |
| Thixogel | 6 | Thixogel | 8 |
| Glyceryl Behenate/Eicosadioate | 4.5 | Glyceryl Behenate/Eicosadioate | 3 |
| Natrosol 250 HRB | 0.65 | Natrosol 250 HRB | 0.5 |
| Glycerin (99.5%) | 2.817 | Disodium hydrogen phosphate | 4 |
| Calcium Nitrate | 5 | Monosodium dihydrogen phosphate | 0.1 |
| Calcium Hypochlorite | 1 | Trisodium Phospshate | 1 |
| Calcium Silicate | 1.5 | Sodium Monofluorophosphate | 1.2 |
| Magensium aluminum silicate colloidal | 3 | Carbopol Aqua SF-1 | 2 |
| Cocamine Oxide (30%) | 5.5 | Polyaminopropyl Biguanide | 1 |
| Sodium Saccharin | 0.083 | Sodium Saccharin | 0.083 |
| Flavor | 1.5 | Acrylates/Ammonium Methacrylate Copolymer | 5 |
| Sodium Chloride | 0.5 | Isopropanol | 16.117 |
| Brushite | 4 | | |
| Calcium Carbonate | 1.35 | | |
| Wintergreen oil | 0.1 | | |
| TiO2 | 1 | | |

example 14: A non-aqueous personal care dental cream / toothpaste, suitable for a dual-chamber device, is manufactured using the following ingredients.

| Ca-Substrate | wt.-% | P-Substrate | wt.-% |
|---|---|---|---|
| Polyisobutene | 2 | Polyisobutene | 2 |
| Polyisodecene | 55.317 | Polyisodecene | 51 |
| Syloid AL-1FP | 2 | Syloid AL-1FP | 2 |
| Syloblanc 34 | 6 | Syloblanc 34 | 6 |
| Thixogel | 8 | Thixogel | 7 |
| Glyceryl Behenate/Eicosadioate | 3 | Glyceryl Behenate/ Eicosadioate | 3 |
| Calcium Propionate | 5 | Polyglyceryl-10 Behenate/Eicosadioate | 1.5 |
| Calcium Hydroxide | 0.5 | Disodium hydrogen phosphate | 4 |
| Calcium Silicate | 1.5 | Monosodium dihydrogen phosphate | 0.1 |
| Calcium Peroxide | 1 | Trisodium Phospshate | 1 |
| Calcium Perborate | 1 | Sodium Monofluorophos-phate | 1.2 |
| Magensium aluminum silicate colloidal | 2 | Carbopol Aqua SF-1 | 1 |
| Cocamine Oxide (30%) | 4.5 | Polyaminopropyl Biguanide | 1 |
| Sodium Saccharin | 0.083 | Sodium Saccharin | 0.083 |
| Flavor | 1.5 | Acrylates/Ammonium Methacrylate Copolymer | 5 |
| Sodium Chloride | 0.5 | Isopropanol | 14.117 |
| Brushite | 4 | | |
| Calcium Carbonate | 1 | | |
| Wintergreen oil | 0.1 | | |
| TiO2 | 1 | | |

Other examples for personal care dental cream / toothpaste may be created by any combination of a Ca- and a P-substrate from the cited examples 12 - 14.

example 15: A professional care product, suitable for a dual-chamber device, is manufactured using the following ingredients.

### example 15.1

| Ca-Substrate | wt.-% | P-Substrate | wt.-% |
|---|---|---|---|
| Water | 21.067 | Water | 10 |
| Sorbitol (70%) | 41 | Polyisodecene | 10 |
| Glycerin | 3.933 | Propylene Glycol | 20 |
| Syloblanc 34 | 9 | Syloblanc 34 | 6 |
| Guar gum | 2 | Thixogel | 5 |
| Calcium Nitrate | 15 | Glyceryl Behenate / Eicosadioate | 3.25 |
| Calcium Chloride | 5 | Polyglyceryl-10 Behenate/ Eicosadioate | 4 |
| Calcium Silicate | 3 | NaClO2 | 10 |
| | | Na3PO4 | 2 |
| | | Na2HPO4 | 17.5 |
| | | NaH2PO4 | 0.25 |
| | | NaF | 5 |
| | | Acrylates/Ammonium Methacrylate Copolymer | 5 |
| | | Carbopol Aqua SF-1 | 2 |

### example 15.2

| Ca-Substrate | wt.-% | P-Substrate | wt.-% |
|---|---|---|---|
| Polyisobutene | 15 | Water | 10 |
| Polyisodecene | 15 | Polyisodecene | 9 |
| Propylene Glycol | 20 | Polyisobutene | 5 |
| Syloblanc 34 | 6 | Propylene Glycol | 20 |
| Thixogel | 8 | Syloblanc 34 | 6 |
| Glyceryl Behenate/Eicosadioate | 6 | Thixogel | 5 |
| Polyglyceryl-10 Behenate/Eicosadioate | 6 | Glyceryl Behenate/ Eicosadioate | 3.25 |
| Calcium Propionate | 5 | Polyglyceryl-10 Behenate/ Eicosadioate | 8 |
| Calcium Peroxide | 15 | NaClO2 | 2 |
| Calcium Silicate | 4 | Na3PO4 | 2 |
| | | Na2HPO4 | 17.5 |
| | | NaH2PO4 | 0.25 |
| | | NaF | 5 |
| | | Acrylates/ Ammonium Methacrylate Copolymer | 5 |
| | | Carbopol Aqua SF-1 | 2 |

### example 15.3

| Ca-Substrate | wt.-% | P-Substrate | wt.-% |
|---|---|---|---|
| Water | 18 | Polyisobutene | 8.88 |
| Polyisodecene | 20 | Polyisodecene | 12 |
| Polyisobutene | 15 | Propylene Glycol | 20 |
| Syloblanc 34 | 6 | Syloblanc 34 | 5 |
| Thixogel | 8 | Thixogel | 4 |
| Glyceryl Behenate/Eicosadioate | 8 | Glyceryl Behenate/ Eicosadioate | 5 |
| Polawax | 6 | Polyglyceryl-10 Behenate/ Eicosadioate | 8 |
| Calcium Propionate | 15 | Disodium hydrogen phosphate | 0.12 |
| Calcium Silicate | 4 | Monosodium dihydrogen phosphate | 12 |
| | | NaF | 3 |
| | | Carbamide Peroxide | 15 |
| | | Acrylates/ Ammonium Methacrylate Copolymer | 5 |
| | | Carbopol Aqua SF-1 | 2 |

example 16: A professional care product (dual snap use of Tooth cream), suitable for a dual-chamber device, is manufactured using the following ingredients.

### ex. 16.1

| Ca-Substrate | wt.-% | P-Substrate | wt.-% |
|---|---|---|---|
| Polyisobutene | 11.417 | Water | 17.13 |
| Polyisodecene | 37 | Polyisodecene | 26 |
| Propylene Glycol | 20 | Syloid AL-1FP | 2 |
| Syloblanc 34 | 3 | Syloblanc 34 | 6 |
| Thixogel | 3 | Thixogel | 8 |
| Glyceryl Behenate/Eicosadioate | 4 | Glyceryl Behenate/Eicosadioate | 3 |
| Polyglyceryl-10 Behenate/Eicosadioate | 6 | Natrosol 250 HRB | 0.5 |
| Calcium Propionate | 5 | Disodium hydrogen phosphate | 1.6 |
| Calcium Peroxide | 5 | Monosodium dihydrogen phosphate | 8.07 |
| Calcium Silicate | 2 | Sodium Monofluorophosphate | 3.5 |
| TiO2 | 1 | Carbopol Aqua SF-1 | 2 |
| Polysorbate 20 | 2 | Polyaminopropyl Biguanide | 1 |
| Flavor | 0.5 | Sodium Saccharin | 0.083 |
| Sodium Saccharin | 0.083 | Acrylates/Ammonium Methacrylate Copolymer | 5 |
| | | Isopropanol | 16.117 |

### example 16.2

| Ca-Substrate | wt.-% | P-Substrate | wt.-% |
|---|---|---|---|
| Water | 30.867 | Water | 20 |
| Polyisodecene | 10 | Polyisodecene | 26.917 |
| Silicon oil | 6 | Syloblanc 34 | 6 |
| Propylene Glycol | 5 | Thixogel | 5 |
| Glycerin (99.5%) | 10 | Glyceryl Behenate/Eicosadioate | 3 |
| Syloblanc 34 | 8 | Polyglyceryl-10 Behenate/ Eicosadioate | 4 |
| Syloid AL-1FP | 2 | Natrosol 250 HRB | 0.5 |
| Thixogel | 5 | Disodium hydrogen phosphate | 4.9 |
| Guar gum | 1 | Monosodium dihydrogen phosphate | 0.1 |
| Natrosol 250 MRB | 0.75 | Sodium Monofluorophosphate | 1.5 |
| Glyceryl Behenate/Eicosadioate | 4 | Carbopol Aqua SF-1 | 2 |
| Polyglyceryl-10 Behenate/Eicosadioate | 4 | Polyaminopropyl Biguanide | 1 |
| Calcium Nitrate | 5 | Sodium Saccharin | 0.083 |
| Calcium Silicate | 2 | Acrylates/Ammonium Methacrylate Copolymer | 5 |
| TiO2 | 1 | Isopropanol | 20 |
| Cocamine Oxide | 4.5 | | |
| Flavor | 0.8 | | |
| Sodium Saccharin | 0.083 | | |

The disclosed examples showed a bimodal, simultaneous effect when applied to the teeth: Bleaching / cleaning the stain from the teeth and re-mineralizing of the teeth were observed. As a result a person applying the inventive oral care composition obtains healthier and brighter teeth, when compared with known oral care compositions.

## Claims

1. An oral care composition consisting of a first and a second composition, wherein
• said first and second composition being maintained separate from each other until dispensed and combined for simultaneous application; and
• said first composition contains a phosphate ion releaser having a water solubility of at least 2.1 g/l (20°C); and
• said second composition contains a calcium ion releaser having a water solubility of at least 2.1 g/l (20°C); and
• said first and / or said second composition contains a film forming agent; and
• said first and / or said second composition contains an acrylate polymer ion releaser; and
• said first and / or said second composition contains a silicate ion releaser; and
• said first and / or said second composition optionally contains abrasive particles; and
• said first composition optionally contains a fluoride ion releaser.

2. The composition according to claim 1, wherein said phosphate ion releaser has a water solubility of at least 2.1 g/l (20°C) and is selected from the group consisting of alkali metal-, urea-, ammonium-phosphates.

3. The composition according to any of claims 1 - 2,
wherein said calcium ion releaser has a water solubility of at least 2.1 g/l (20°C) and is selected from the group consisting of calcium nitrate, calcium oxide, calcium hydroxide, calcium bromide, calcium peroxide, calcium formate, calcium acetate, calcium malate, Calcium citrate malate, calcium chloride, calcium glycerophosphate, Calcium propionate, calcium acetate, calcium hypochlorite, Calcium lactate gluconate, calcium benzoate, calcium fumarate, calcium lactate, calcium butyrate, calcium isobutyrate, calcium maleate, calcium valerate.

4. The composition according to any of claims 1 - 3, wherein said film forming agent is selected from the group consisting of poly(meth)acrylate polymers.

5. The composition according to any of claims 1 - 4, wherein said silicate ion releaser is selected from the group consisting of alkali silicates and / or calcium silicates.

6. The composition according to any of claims 1 - 5, wherein said abrasive particles have an average particle size of 50 - 200 µm.

7. The composition according to any of claims 1 - 6, wherein said fluorid ion releaser is selected from the group consisting of alkali metal fluorides, tin fluoride, indium fluoride, zirconium fluoride, copper fluoride, nickel fluoride, palladium fluoride, fluorozirconates, fluorosilicates, fluoroborates, fluorostannites, organic fluorides, alkali metal monofluorophosphates, ammonium monofluorophosphate, aluminium monofluorophosphate.

8. The composition according to any of claims 1 - 7, wherein said first and / or said second composition further contains one or more ingredients independently selected from the group consisting of humectants, thickening agents / binding agents, detergents, colorants, perfumes, flavouring agents / sweeteners, pH regulators, nutritients, preservatives, bleaching agents and carriers.

9. The composition according to any of claims 1 - 8
• having a pH of at least 7, and/or
• which contains less than 5 wt.-% of water.

10. The composition according to any of claims 1 - 9 which is either a dental cream or an oral solution.

11. The composition according to any of claims 1 - 10, wherein no Carboxymethylcellulose salts are present.

12. A method for manufacturing an oral care composition according to any of claims 1 - 11, comprising the steps of
i) manufacturing a composition containing all ingredients of said oral care composition, except the calcium ion releaser, phosphate ion releaser and fluoride ion releaser(if present);
ii) dividing the thus obtained composition into two parts;
iii)adding one or more calcium ion releaser to one of said parts;
iv) adding one or more phosphate ion releaser and fluoride ion releaser (if present) to the other part and
v) filling the thus obtained first and second composition into a two compartment device.

13. A dental cream according to claim 10 for use in a method of cleaning the teeth of a subject in need thereof comprising the step of brushing the teeth with an electrical tooth brush.

14. A Composition according to any of claims 1 - 11 as pharmaceutical.

15. A Composition according to any of claims 1 - 11 for use in the treatment, prevention and or delay of progression of disorders related to loss of material of the teeth.

## Patentansprüche

1. Eine Mundpflegezusammensetzung, bestehend aus einer ersten und einer zweiten Zusammensetzung, wobei
• besagte erste und zweite Zusammensetzung voneinander getrennt gehalten werden bis sie abgegeben und kombiniert werden zur simultanen Anwendung; und
• besagte erste Zusammensetzung einen Phosphat - Ion - Abgeber mit einer Wasserlöslichkeit von zumindest 2.1 g/l (20°C)enthält; und
• besagte zweite Zusammensetzung einen Kalzium - Ionen
- Abgeber mit einer Wasserlöslichkeit von zumindest 2.1 g/l (20°C) enthält; und
• besagte erste und / oder besagte zweite Zusammensetzung ein filmbildendes Agens enthält; und
• besagte erste und / oder besagte zweite Zusammensetzung einen Acrylat - Polymer - Ionen - Abgeber enthält; und
• besagte erste und / oder besagte zweite Zusammensetzung einen Silikat - Ionen - Abgeber enthält; und
• besagte erste und / oder besagte zweite Zusammensetzung optional abrasive Partikel enthält; und
• besagte erste Zusammensetzung optional einen Fluorid - Ionen - Abgeber enthält.

2. Die Zusammensetzung nach Anspruch 1, Phosphat - Ion - Abgeber eine Wasserlöslichkeit von zumindest 2.1 g/l (20°C) aufweist und aus der Gruppe umfassend Alkalimetall-, Harnstoff- und Ammonium- Phosphate ausgewählt ist.

3. Die Zusammensetzung nach irgendeinem der Ansprüche 1 - 2, wobei besagter Kalzium - Ionen - Abgeber eine Wasserlöslichkeit von zumindest 2.1 g/l (20°C) aufweist und aus der Gruppe umfassend Kalziumnitrat, Kalziumoxid, Kalziumhydroxid, Kalziumbromid, Kalziumperoxid, Kalziumformat, Kalziumazetat, Kalziummalat, Kalziumcitrat-malat, Kalziumchlorid, Kalziumglycerophosphat, Kalziumpropionat, Kalziumacetat, Kalziumhypochlorit, Kalziumlactate-gluconat, Kalziumbenzoat, Kalziumfumarat, Kalziumlaktat, Kalziumbutyrat, Kalziumisobutyrat, Kalziummaleate und Kalziumvalerate ausgewählt ist.

4. Die Zusammensetzung nach irgendeinem der Ansprüche 1 - 3, wobei besagtes filmbildendes Agens aus der Gruppe umfassend die Poly(meth)acrylat - Polymere ausgewählt ist.

5. Die Zusammensetzung nach irgendeinem der Ansprüche 1 - 4, wobei besagter Silikat - Ionen Abgeber aus der Gruppe umfassend Alkalisilikate und / oder Kalziumsilikate ausgewählt ist.

6. Die Zusammensetzung nach irgendeinem der Ansprüche 1 - 5, wobei die besagten abrasiven Partikel eine mittlere Partikelgrösse von 50 - 200 µm aufweisen.

7. Die Zusammensetzung nach irgendeinem der Ansprüche 1 - 6, wobei besagter Fluorid - Ionen - Abgeber ausgewählt ist aus der Gruppe umfassend Alkalimetall - Fluoride, Zinnfluorid, Indiumfluorid, Zirkoniumfluorid, Kupferfluorid, Nickelfluorid, Palladiumfluorid, Fluorozirkonate, Fluorosilikate, Fluoroborate, Fluorostannate, organische Fluoride, Alkalimetall - Monofluorophosphate, Ammonium - Monofluorophosphate und Aluminium - Monofluorophosphate ausgewählt ist.

8. Die Zusammensetzung nach irgendeinem der Ansprüche 1 - 7, wobei besagte erste und / oder besagte zweite Zusammensetzung weiterhin einen oder mehrere Ingredienzien enthält, unabhängig voneinander ausgewählt aus der Gruppe umfassend Feuchthaltemittel, verdickende Agentien / bindende Agentien, Reinigungsmittel, Farbstoffe, Parfüme, geschmacksgebende Agentien / Süssstoffe, pH Regulierer, Nährstoffe, Konservierungsstoffe, bleichende Agentien und Trägerstoffe.

9. Die Zusammensetzung nach irgendeinem der Ansprüche 1 - 8
• mit einem pH - wert von zumindest 7, und/oder
• welche weniger als 5 wt.-% Wasser enthält.

10. Die Zusammensetzung nach irgendeinem der Ansprüche 1 - 9 welche entweder eine Zahncreme oder eine Mundspülung ist.

11. Die Zusammensetzung nach irgendeinem der Ansprüche 1 - 10, wobei keine Carboxymethyl- Zellulose Saltze anwesend sind.

12. Ein Verfahren zur Herstellung einer Mundpflegezusammensetzung nach irgendeinem der Ansprüche 1 - 11, umfassend die Schritte
i) Herstellen einer Zusammensetzung welche alle Bestandteile besagter Mundpflegezusammensetzung enthält, mit Ausnahme des Kalzium - Ionen - Abgebers, Phosphat - Ion - Abgebers und Fluorid - Ionen - Abgebers (sofern anwesend);
ii) Teilen der so erhaltenen Zusammensetzung in zwei Teile;
iii)Zugabe von einem oder mehreren Kalzium - Ionen - Abgebern zu einem der besagten Teile;
iv) Zugabe von einem oder mehreren Phosphat - Ionen - Abgebern und Fluorid - Ionen - Abgebern (sofern anwesend) zu dem anderen Teil und
v) Abfüllen der so erhaltenen ersten und zweiten Zusammensetzung in eine Zweikammervorrichtung.

13. Eine Methode zum Reinigen von Zähnen, in einem Subjekt das dessen bedarf, umfassend den Schritt des Bürstens der Zähne mit einer elektrischen Zahnbürste unter Verwendung eine Zahncreme nach Anspruch 10.

14. Eine Zusammensetzung nach irgendeinem der Ansprüche 1 - 11 als Arzneimittel.

15. Eine Zusammensetzung nach irgendeinem der Ansprüche 1 - 11 zur Verwendung in der Behandlung, Vorbeugung, oder der Verzögerung des Fortschreitens von Krankheiten im Zusammenhang mit dem Verlust von Material an den Zähnen.

## Revendications

1. Une composition de soin buccal consistant d'une première et d'une deuxième composition,
• ladite première et deuxième composition étant maintenues séparément l'une de l'autre jusqu'à leur distribution et combinaison pour une application simultané; et
• ladite première composition contenant un donneur d'ion phosphate avec une solubilité dans l'eau d'au moins 2.1 g/l (20°C); et
• ladite deuxième composition contenant un donneur d'ion calcium avec une solubilité dans l'eau d'au moins 2.1 g/l (20°C); et
• ladite première et/ou deuxième composition contenant un agent filmogène; et
• ladite première et/ou deuxième composition contenant un donneur d'ion polymère acrylate; et
• ladite première et/ou deuxième composition contenant un donneur d'ion silicate; et
• ladite première et/ou deuxième composition contenant des particules abrasives; et
• ladite première composition optionnellement contenant un donneur d'ion fluorure.

2. La composition selon la revendication 1, ledit donneur d'ion phosphate ayant une solubilité dans l'eau d'au moins 2.1 g/l (20°C) et étant sélectionné du groupe consistant des phosphates de métal alcaline, d'urée, d'ammonium.

3. La composition selon l'une des revendications 1 - 2, ledit donneur d'ion calcium ayant une solubilité dans l'eau d'au moins 2.1 g/l (20°C) et étant sélectionné du groupe consistant de nitrate de calcium, oxyde de calcium, hydroxyde de calcium, bromure de calcium, peroxyde de calcium, formiate de calcium, acétate de calcium, malate de calcium, citrate malate de calcium, chlorure de calcium, glycérophosphate de calcium, propionate de calcium, acétate de calcium, hypochlorite de calcium, lactate gluconate de calcium, benzoate de calcium, fumarate de calcium, lactate de calcium, butyrate de calcium, isobutyrate de calcium, maleate de calcium, valérate de calcium.

4. La composition selon l'une des revendications 1 - 3, ledit agent filmogène étant sélectionné du groupe consistant de polymères poly(méth)acrylate.

5. La composition selon l'une des revendications 1 - 4, ledit donneur d'ion silicate étant sélectionné du groupe consistant de silicates alcalins et/ou silicates de calcium.

6. La composition selon l'une des revendications 1 - 5, lesdites particules abrasives ayant une largeur moyenne de particule comprise entre 50 - 200 µm.

7. La composition selon l'une des revendications 1 - 6, ledit donneur d'ion fluorure étant sélectionné du groupe consistant de fluorures de métal alcalin, fluorure d'étain, fluorure d'indium, fluorure de zirconium, fluorure de cuivre, fluorure de nickel, fluorure de palladium, fluorozirconates, fluorosilicates, fluoroborates, fluorostannites, fluorures organiques, monofluorophosphates de métal alcalin, monofluorophosphate d'ammonium, monofluorophosphate d'aluminium.

8. La composition selon l'une des revendications 1 - 7, ladite première et/ou deuxième composition en outre contenant un ou plusieurs ingrédients indépendamment sélectionnés du groupe consistant de humectants, agents épaississants / agents liants, détergents, colorants, parfumes, agents aromatisants / édulcorants, régulateurs de pH, nutriments, préservatives, agents blanchissants et porteurs.

9. La composition selon l'une des revendications 1 - 8
• ayant un pH d'au moins 7, et/ou
• contenant moins de 5 % par poids d'eau.

10. La composition selon l'une des revendications 1 - 9 étant une crème dentale ou une solution orale.

11. La composition selon l'une des revendications 1 - 10, aucuns sels de carboxyméthylcellulose étant présents.

12. Un procédé de fabrication d'une composition de soin buccal selon l'une des revendications 1 - 11, comprenant les étapes
i) de fabrication d'une composition contenant tous ingrédients de ladite composition de soin buccal, sauf le donneur d'ion calcium, le donneur d'ion phosphate et le donneur d'ion fluorure (si présent);
ii) de diviser la composition ainsi obtenue en deux parties;
iii) d'additionner un ou plusieurs donneurs d'ion calcium à une des parties;
iv) d'additionner un ou plusieurs donneurs d'ion phosphate et donneurs d'ion fluorure (si présents) à l'autre partie et
v) de remplir ladite première et deuxième composition ainsi obtenues dans un dispositif à deux compartiments.

13. Une crème dentifrice selon la revendication 10 pour l'utilisation dans un procédé de nettoyage de dents d'un sujet en ayant besoin, comprenant l'étape de brosser les dents avec une brosse de dents électrique.

14. Une composition selon l'une des revendications 1 - 11 comme pharmaceutique.

15. Une composition selon l'une des revendications 1 - 11 pour l'utilisation dans le traitement, prévention et/ou retard de la progression des désordres attribuable à la perte de matériau des dents.
